# EUROPÄISCHE PATENTSCHRIFT

(11) **EP 1 523 493 B1**
(45) Veröffentlichungstag und Bekanntmachung des Hinweises auf die Patenterteilung: **04.09.2013**
(21) Anmeldenummer: 03762676.9
(22) Anmeldetag: 09.07.2003
(51) Int. Cl.: C07K 5/10, C07D 417/12, A61K 38/07, A61K 31/4523, A61P 35/00

(54) **NEUE TUBULYSINANALOGA**
NOVEL TUBULYSIN ANALOGUES
NOUVEAUX ANALOGUES DE TUBULYSINE

(30) Priorität: 09.07.2002 DE 10230874; 13.11.2002 DE 10252719
(43) Veröffentlichungstag der Anmeldung: 20.04.2005
(73) Patentinhaber: Dömling, Alexander, 80797 München (DE)
(72) Erfinder: DOEMLING, Alexander, 81243 München (DE); HENKEL, Bernd, 82152 Planegg (DE); BECK, Barbara, 82140 Olching (DE); ILLGEN, Katrin, 82152 Planegg (DE); SAKAMURI, Sukumar, Plainsboro, NJ 08536 (US); MENON, Sanjay, Plainsboro, NJ 08536 (US)
(74) Vertreter: Forstmeyer, Dietmar
(86) Internationale Anmeldenummer: PCT/EP2003/007419
(87) Internationale Veröffentlichungsnummer: WO 2004/005327

(56) Entgegenhaltungen:
- WO-A2-2004/046170
- DE-A- 10 008 089

## Beschreibung

Die Vorliegende Erfindung betrifft neue Tubulysinanaloga sowie die Verwendung dieser Verbindungen zur Behandlung von Krebserkrankungen.

Die Tubulysine wurden erstmals von der Gruppe von Höfle und Reichenbach (GBF Braunschweig) aus einer Kulturbrühe von Stämmen des Myxobakteriums *Archangium gephyra* isoliert (F. Sasse et al. J. Antibiot. 2000, 53, 879-885; WO9813375; DE 10008089). Diese Verbindungen haben eine ausgesprochen hohe cytotoxische Aktivität gegenüber Säugetierzellinien mit IC₅₀-Werten im picomolaren Bereich und sind daher als potentielle Krebsmedikamente von grossem Interesse. Tubulysine (I) sind Tetrapeptide, die drei ungewöhnliche Aminosäuren enthalten, was ihre Synthese zu einer Herausforderung für die organische Synthesechemie macht.

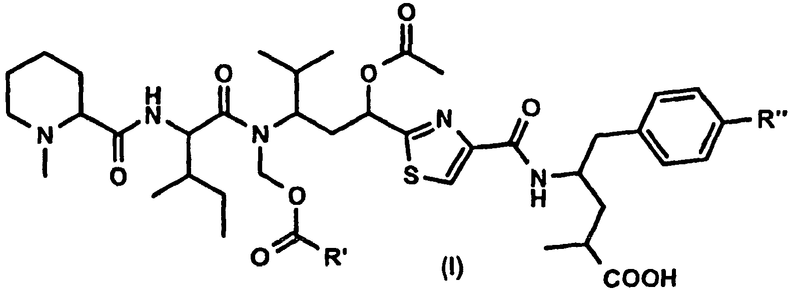

| | |
|---|---|
| Tubulysin A: R' = CH₂CH(CH₃)₂; | R'' = OH |
| Tubulysin B: R' = CH₂CH₂CH₃; | R'' = OH |
| Tubulysin C: R' = CH₂CH₃; | R'' = OH |
| Tubulysin D: R' = CH₂CH(CH₃)₂; | R'' = H |
| Tubulysin E: R' = CH₂CH₂CH₃; | R'' = H |
| Tubulysin F: R' = CH₂CH₃; | R'' = H |

Ziel der vorliegenden Erfindung war es, neue Tubulysinanaloga bereitzustellen, die eine höhere Wirksamkeit bzw. bessere pharmakologische Eigenschaften als die Naturstoffe aufweisen.

Die vorliegende Erfindung betrifft Verbindungen der allgemeinen Formel (II)
wobei
Y ein Sauerstoffatom, ein Schwefelatom oder eine Gruppe der Formel NR¹⁶ ist, wobei R¹⁶ ein Wasserstoffatom, ein Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest ist;
R¹ eine C₁-C₄ Alkylgruppe ist;
R² und R³ zusammen eine Gruppe der Formel (CH₂)ₙ mit n gleich 2, 3, 4 oder 5 sind;
R⁴ ein Wasserstoffatom oder eine Methylgruppe ist;
R⁶ eine C₁-C₆ Alkyl-, eine C₃-C₆ Cycloalkyl oder eine C₄-C₇ Alkylcycloalkylgruppe ist;
R⁷ ein Wasserstoffatom oder eine Methylgruppe ist;
R⁸ ein Wasserstoffatom, ein Alkyl-, Alkenyl- oder ein Aralkylrest ist;
R⁹ eine C₁-C₆ Alkylgruppe ist;
R¹⁰ ein Wasserstoffatom oder eine Methylgruppe ist;
R¹¹ ein Wasserstoffatom, ein Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest ist;
R¹⁸ ein Wasserstoffatom oder eine Methylgruppe ist;
R²¹ eine OH, NH₂, Alkyloxy-, Alkylamino oder eine Dialkylaminogruppe ist;
R²² ein Halogenatom, eine OH, NO₂, NH₂, Alkyloxy-, Alkylamino oder eine Dialkylaminogruppe ist und
p gleich 0, 1, 2 oder 3 ist, 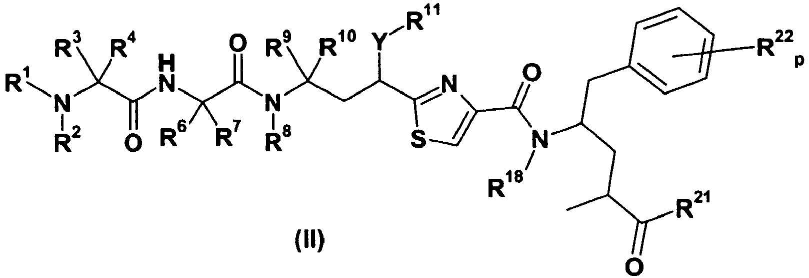 oder ein pharmakologisch akzeptables Salz, Solvat, Hydrat oder eine pharmakologisch akzeptable Formulierung derselben, wobei die folgende Verbindung ausgenommen ist:

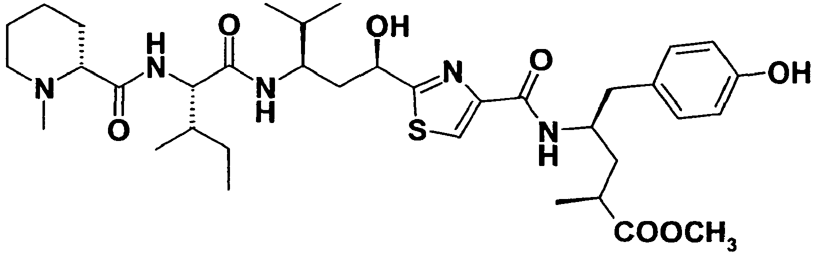

Der Ausdruck Alkyl oder Alk bezieht sich auf eine gesättigte, geradkettige oder verzweigte Kohlenwasserstoffgruppe, die 1 bis 20 Kohlenstoffatome, vorzugsweise 1 bis 12 Kohlenstoffatome, besonders bevorzugt 1 bis 6 Kohlenstoffatome aufweist, z.B. die Methyl-, Ethyl-, Propyl-, Isopropyl-, Isobutyl-, n-Butyl-, tert-Butyl, n-Hexyl-, 2,2-Dimethylbutyl- oder n-Octyl-Gruppe.

Die Ausdrücke Alkenyl und Alkinyl beziehen sich auf zumindest teilweise ungesättigte, geradkettige oder verzweigte Kohlenwasserstoffgruppen, die 2 bis 20 Kohlenstoffatome, vorzugsweise 2 bis 12 Kohlenstoffatome, besonders bevorzugt 2 bis 6 Kohlenstoffatome aufweisen, z. B. die Ethenyl-, Allyl-, Acetylenyl-, Propargyl-, Isoprenyl- oder Hex-2-enyl-Gruppe. Bevorzugt weisen Alkenylgruppen eine oder zwei (besonders bevorzugt eine) Doppelbindungen bzw. Alkinylgruppen eine oder zwei (besonders bevorzugt eine) Dreifachbindungen auf.

Des weiteren beziehen sich die Begriffe Alkyl, Alkenyl und Alkinyl auf Gruppen, bei der ein oder mehrere Wasserstoffatome durch ein Halogenatom (bevorzugt F oder Cl) ersetzt sind wie z. B. die 2,2,2-Trichlorethyl-, oder die Trifluormethylgruppe.

Der Ausdruck Heteroalkyl bezieht sich auf eine Alkyl-, eine Alkenyl- oder eine Alkinyl-Gruppe, in der ein oder mehrere (bevorzugt 1, 2 oder 3) Kohlenstoffatome durch ein Sauerstoff-, Stickstoff-, Phosphor-, Bor-, Selen-, Silizium- oder Schwefelatom ersetzt sind (bevorzugt Sauerstoff, Schwefel oder Stickstoff). Der Ausdruck Heteroalkyl bezieht sich des weiteren auf eine Carbonsäuregruppe oder eine von einer Carbonsäure abgeleitete Gruppe wie z. B. Acyl (Alkyl-CO-), Acylalkyl, Alkoxycarbonyl, Acyloxy, Acyloxyalkyl, Carboxyalkylamid oder Alkoxycarbonyloxy.

Beispiele für Heteroalkylgruppen sind Gruppen der Formeln R^{a}-O-Y^{a}-, R^{a}-S-Y^{a}-, R^{a}-N(R^{b}) -Y^{a}-, R^{a}-CO-Y^{a}-, R^{a}-O-CO-Y^{a}-, R^{a}-CO-O-Y^{a}-, R^{a}-CO-N(R^{b}) -Y^{a}-, R^{a}-N(R^{b}) -CO-Y^{a}-, R^{a}-O-CO-N(R^{b})-Y^{a}-, R^{a}-N(R^{b})-CO-O-Y^{a}-, R^{a}-N(R^{b}) -CO-N(R^{c})-Y^{a}-. R^{a}-O-CO-O-Y^{a}-, R^{a}-N(R^{b})-C(=NR^{d}) -N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b}) -Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}- , R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wobei R^{a} ein Wasserstoffatom, eine C₁-C₆-Alkyl-, eine C₂-C₆-Alkenyl-oder eine C₂-C₆-Alkinylgruppe; R^{b} ein Wasserstoffatom, eine C₁-C₆-Alkyl-, eine C₂-C₆-Alkenyl- oder eine C₂-C₆-Alkinylgruppe; R^{c} ein Wasserstoffatom, eine C₁-C₆-Alkyl-, eine C₂-C₆-Alkenyl- oder eine C₂-C₆-Alkinylgruppe; R^{d} ein Wasserstoffatom, eine C₁-C₆-Alkyl-, eine C₂-C₆-Alkenyl-oder eine C₂-C₆-Alkinylgruppe und Y^{a} eine Bindung, eine C₁-C₆-Alkylen-, eine C₂-C₆-Alkenylen- oder eine C₂-C₆-Alkinylengruppe ist, wobei jede Heteroalkylgruppe mindestens ein Kohlenstoffatom enthält und ein oder mehrere Wasserstoffatome durch Fluor- oder Chloratome ersetzt sein können. Konkrete Beispiele für Heteroalkylgruppen sind Methoxy, Trifluormethoxy, Ethoxy, n-Propyloxy, iso-Propyloxy, tert-Butyloxy, Methoxymethyl, Ethoxymethyl, Methoxyethyl, Methylamino, Ethylamino, Dimethylamino, Diethylamino, iso-Propylethylamino, Methylaminomethyl, Ethylaminomethyl, Di-iso-Propylaminoethyl, Enolether, Dimethylaminomethyl, Dimethylaminoethyl, Acetyl, Propionyl, Butyryloxy, Acetyloxy, Methoxycarbonyl, Ethoxy-carbonyl, N-Ethyl-N-Methylcarbamoyl oder N-Methylcarbamoyl. Weitere Beispiele für Heteroalkylgruppen sind Nitril-, Isonitril, Cyanat-, Thiocyanat-, Isocyanat-, Isothiocyanat und Alkylnitrilgruppen.

Der Ausdruck Cycloalkyl bezieht sich auf eine gesättigte oder teilweise ungesättigte (z. B. Cycloalkenyl) cyclische Gruppe, die einen oder mehrere Ringe (bevorzugt 1 oder 2) aufweist, die insgesamt 3 bis 14 Ring-Kohlenstoffatome, vorzugsweise 3 bis 10 (insbesondere 3, 4, 5, 6 oder 7) Ring-Kohlenstoffatome enthalten. Der Ausdruck Cycloalkyl bezieht sich weiterhin auf derartige Gruppen, bei denen ein oder mehrere Wasserstoffatome durch Fluor-, Chlor-, Brom- oder Jodatome oder OH, =O, SH, =S, NH₂, =NH oder NO₂-Gruppen ersetzt sind also z. B. cyclische Ketone wie z. B. Cyclohexanon, 2-Cyclohexenon oder Cyclopentanon. Weitere konkrete Beispiele für Cycloalkylgruppen sind die Cyclopropyl-, Cyclobutyl-, Cyclopentyl-, Spiro[4,5]-decanyl-, Norborny-, Cyclohexyl-, Cyclopentenyl-, Cyclohexadienyl-, Decalinyl-, Cubanyl-, Bicyclo[4.3.0]-nonyl-, Tetralin-, Cyclopentylcyclohexyl-, Fluorcyclohexyl- oder die Cyclohex-2-enyl-Gruppe.

Der Ausdruck Heterocycloalkyl bezieht sich auf eine Cycloalkylgruppe wie oben definiert, in der ein oder mehrere (bevorzugt 1, 2 oder 3) Ring-Kohlenstoffatome durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heterocycloalkylgruppe 1 oder 2 Ringe mit 3 bis 10 (insbesondere 3, 4, 5, 6 oder 7) Ringatomen. Der Ausdruck Heterocycloalkyl bezieht sich weiterhin auf derartige Gruppen, bei denen ein oder mehrere Wasserstoffatome durch Fluor-, Chlor-, Brom- oder Jodatome oder OH, =O, SH, =S, NH₂, =NH oder NO₂-Gruppen ersetzt sind. Beispiele sind die Piperidyl-, Morpholinyl-, Urotropinyl-, Pyrrolidinyl-, Tetrahydrothiophenyl-, Tetrahydropyranyl-, Tetrahydrofuryl-, Oxacyclopropyl-, Azacyclopropyl- oder 2-Pyrazolinyl-Gruppe sowie Lactame, Lactone, cyclische Imide und cyclische Anhydride.

Der Ausdruck Alkylcycloalkyl bezieht sich auf Gruppen, die entsprechend den obigen Definitionen sowohl Cycloalkylwie auch Alkyl-, Alkenyl- oder Alkinylgruppen enthalten, z. B. Alkylcycloalkyl-, Alkylcycloalkenyl-, Alkenylcycloalkyl- und Alkinylcycloalkylgruppen. Bevorzugt enthält eine Alkylcycloalkylgruppe eine Cycloalkylgruppe, die einen oder zwei Ringsysteme aufweist, welche insgesamt 3 bis 10 (insbesondere 3, 4, 5, 6 oder 7) Ring-Kohlenstoffatome enthält und eine oder zwei Alkyl-, Alkenyl- oder Alkinylgruppen mit 1 oder 2 bis 6 Kohlenstoffatomen.

Der Ausdruck Heteroalkylcycloalkyl bezieht sich auf Alkylcycloalkylgruppen, wie oben definiert, in der ein oder mehrere (bevorzugt 1, 2 oder 3) Kohlenstoffatome durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heteroalkylcycloalkylgruppe 1 oder 2 Ringsysteme mit insgesamt 3 bis 10 (insbesondere 3, 4, 5, 6 oder 7) Ringatomen und eine oder zwei Alkyl, Alkenyl, Alkinyl oder Heteroalkylgruppen mit 1 oder 2 bis 6 Kohlenstoffatomen. Beispiele derartiger Gruppen sind Alkylheterocycloalkyl, Alkylheterocycloalkenyl, Alkenylheterocycloalkyl, Alkinylheterocycloalkyl, Heteroalkylcycloalkyl, Heteroalkylheterocycloalkyl und Heteroalkylheterocylcloalkenyl, wobei die cyclischen Gruppen gesättigt oder einfach, zweifach oder dreifach ungesättigt sind.

Der Ausdruck Aryl bzw. Ar bezieht sich auf eine aromatische Gruppe, die einen oder mehrere Ringe hat, welche insgesamt 6 bis 14 Ring-Kohlenstoffatome, vorzugsweise 6 bis 10 (insbesondere 6) Ring-Kohlenstoffatome enthalten. Der Ausdruck Aryl (bzw. Ar) bezieht sich weiterhin auf derartige Gruppen, bei denen ein oder mehrere Wasserstoffatome durch Fluor-, Chlor-, Brom- oder Jodatome oder OH, SH, NH₂, oder NO₂-Gruppen ersetzt sind. Beispiele sind die Phenyl-, Naphthyl-, Biphenyl-, 2-Fluorphenyl, Anilinyl-, 3-Nitrophenyl oder 4-Hydroxy-phenyl-Gruppe.

Der Ausdruck Heteroaryl bezieht sich auf eine aromatische Gruppe, die einen oder mehrere Ringe hat, welche insgesamt 5 bis 14 Ringatome, vorzugsweise 5 bis 10 (insbesondere 5 oder 6) Ringatome enthalten und ein oder mehrere (bevorzugt 1, 2, 3 oder 4) Sauerstoff-, Stickstoff-, Phosphor- oder Schwefel-Ringatome (bevorzugt O, S oder N) enthält. Der Ausdruck Heteroaryl bezieht sich weiterhin auf derartige Gruppen, bei denen ein oder mehrere Wasserstoffatome durch Fluor-, Chlor-, Brom- oder Jodatome oder OH, SH, NH₂, oder NO₂-Gruppen ersetzt sind. Beispiele sind 4-Pyridyl-, 2-Imidazolyl-, 3-Phenylpyrrolyl-, Thiazolyl-, Oxazolyl-, Triazolyl-, Tetrazolyl-, Isoxazolyl-, Indazolyl-, Indolyl-, Benzimidazolyl-, Pyridazinyl-, Chinolinyl-, Purinyl-, Carbazolyl-, Acridinyl-, Pyrimidyl-, 2,3'-Bifuryl-, 3-Pyrazolyl- und Isochinolinyl-Gruppen.

Der Ausdruck Aralkyl bezieht sich auf Gruppen, die entsprechend den obigen Definitionen sowohl Aryl- wie auch Alkyl-, Alkenyl-, Alkinyl- und/oder Cycloalkylgruppen enthalten, wie z. B. Arylalkyl-, Arylalkenyl-, Arylalkinyl-, Arylcycloalkyl-, Arylcycloalkenyl-, Alkylarylcycloalkyl- und Alkylarylcycloalkenylgruppen. Konkrete Beispiele für Aralkyle sind Toluol, Xylol, Mesitylen, Styrol, Benzylchlorid, o-Fluortoluol, 1H-Inden, Tetralin, Dihydronaphthaline, Indanon, Phenylcyclopentyl, Cumol, Cyclohexylphenyl, Fluoren und Indan. Bevorzugt enthält eine Aralkylgruppe ein oder zwei aromatische Ringsysteme (1 oder 2 Ringe) mit insgesamt 6 bis 10 Ring-Kohlenstoffatomen und ein oder zwei Alkyl-, Alkenyl- und/oder Alkinylgruppen mit 1 oder 2 bis 6 Kohlenstoffatomen und/oder eine Cycloalkylgruppe mit 5 oder 6 Ringkohlenstoffatomen.

Der Ausdruck Heteroaralkyl bezieht sich auf eine Aralkylgruppe wie oben definiert, in der ein oder mehrere (bevorzugt 1, 2, 3 oder 4) Kohlenstoffatome durch ein Sauerstoff-, Stickstoff-, Silizium-, Selen-, Phosphor-, Bor- oder Schwefelatom (bevorzugt Sauerstoff, Schwefel oder Stickstoff) ersetzt sind, d. h. auf Gruppen, die entsprechend den obigen Definitionen sowohl Aryl- bzw. Heteroaryl- wie auch Alkyl-, Alkenyl-, Alkinyl- und/oder Heteroalkyl- und/oder Cycloalkyl- und/oder Heterocycloalkylgruppen enthalten. Bevorzugt enthält eine Heteroaralkylgruppe ein oder zwei aromatische Ringsysteme (1 oder 2 Ringe) mit insgesamt 5 oder 6 bis 10 RingKohlenstoffatomen und ein oder zwei Alkyl-, Alkenyl-und/oder Alkinylgruppen mit 1 oder 2 bis 6 Kohlenstoffatomen und/oder eine Cycloalkylgruppe mit 5 oder 6 Ringkohlenstoffatomen, wobei 1, 2, 3 oder 4 dieser Kohlenstoffatome durch Sauerstoff-, Schwefel- oder Stickstoffatome ersetzt sind.

Beispiele sind Arylheteroalkyl-, Arylheterocycloalkyl-, Arylheterocycloalkenyl-, Arylalkylheterocycloalkyl-, Arylalkenylheterocycloalkyl-, Arylalkinylheterocyclo-alkyl-, Arylalkylheterocycloalkenyl-, Heteroarylalkyl-, Heteroarylalkenyl-, Heteroarylalkinyl-, Heteroarylheteroalkyl-, Heteroarylcycloalkyl-, Heteroarylcycloalkenyl-, Heteroarylheterocycloalkyl-, Heteroarylheterocycloalken-yl-, Heteroarylalkylcycloalkyl-, Heteroarylalkylheterocycloalkenyl-, Heteroarylheteroalkylcycloalkyl-, Heteroarylheteroalkylcycloalkenyl- und Heteroarylheteroalkylheterocycloalkyl-Gruppen, wobei die cyclischen Gruppen gesättigt oder einfach, zweifach oder dreifach ungesättigt sind. Konkrete Beispiele sind die Tetrahydroisochinolinyl-, Benzoyl-, 2- oder 3-Ethyl-indolyl-, 4-Methylpyridino-, 2-, -3- oder 4-Methoxyphenyl-, 4-Ethoxyphenyl-, 2-, 3- oder 4-Carboxyphenylalkylgruppe.

Die Ausdrücke Cycloalkyl, Heterocycloalkyl, Alkylcycloalkyl, Heteroalkylcycloalkyl, Aryl, Heteroaryl, Aralkyl und Heteroaralkyl beziehen sich auch auf Gruppen, in denen ein oder mehrere Wasserstoffatome solcher Gruppen durch Fluor-, Chlor-, Brom- oder Jodatome oder OH, =O, SH, =S, NH₂, =NH oder NO₂-Gruppen ersetzt sind.

Der Ausdruck "gegebenenfalls substituiert" bezieht sich z.B. auf Gruppen, in denen ein oder mehrere Wasserstoffatome durch Fluor-, Chlor-, Brom- oder Jodatome oder OH, =O, SH, =S, NH₂, =NH oder NO₂-Gruppen ersetzt sind. Dieser Ausdruck bezieht sich weiterhin auf Gruppen, die ausschließlich oder zusätzlich mit unsubstituierten C₁-C₆ Alkyl-, C₂-C₆ Alkenyl-, C₂-C₆ Alkinyl-, C₁-C₆ Heteroalkyl-, C₃-C₁₀ Cycloalkyl-, C₂-C₉ Heterocycloalkyl-, C₆-C₁₀ Aryl-, C₁-C₉ Heteroaryl-, C₇-C₁₂ Aralkyl- oder C₂-C₁₁ Heteroaralkyl-Gruppen substituiert sind.

Schutzgruppen sind dem Fachmann bekannt und z. B. in P. J. Kocienski, Protecting Groups, Georg Thieme Verlag, Stuttgart, 1994 sowie in T. W. Greene, P. G. M. Wuts, Protective Groups in Organic Synthesis, John Wiley & Sons, New York, 1999 beschrieben, und sind hier unter Bezugnahme aufgenommen. Gängige Aminoschutzgruppen sind z. B. t-Butyloxycarbonyl- (Boc), Benzyloxycarbonyl- (Cbz, Z), Benzyl- (Bn), Benzoyl- (Bz), Fluorenylmethyloxycarbonyl-(Fmoc), Allyloxycarbonyl -(Alloc), Trichlorethyloxycarbonyl- (Troc), Acetyl- oder Trifluoracetylgruppen.

Verbindungen der Formel (II) können aufgrund ihrer Substitution ein oder mehrere Chiralitätszentren enthalten. Die vorliegende Erfindung umfasst daher sowohl alle reinen Enantiomere und alle reinen Diastereomere, als auch deren Gemische in jedem Mischungsverhältnis. Des weiteren sind von der vorliegenden Erfindung auch alle cis/trans-Isomeren der Verbindungen der allgemeinen Formel (II) sowie Gemische davon umfasst. Des weiteren sind von der vorliegenden Erfindung alle tautomeren Formen der Verbindungen der Formel (II) umfasst.

Bevorzugt ist Y ein Sauerstoffatom.

Des weiteren bevorzugt entspricht R⁶ der Seitenkette von Leucin, Isoleucin oder Valin.

Wiederum bevorzugt ist R¹¹ ein Wasserstoffatom oder eine Gruppe der Formel - (C=O) - (C₁₋₄) Alkyl.

Referenzbeispiele sind Verbindungen der Formel (III), 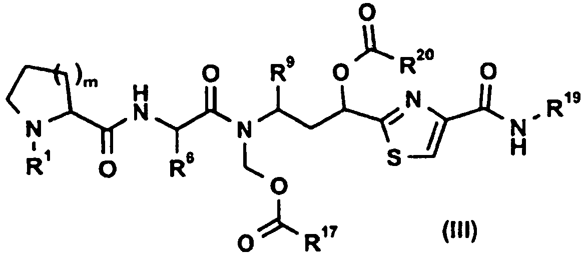 wobei R¹ eine C₁-C₄ Alkylgruppe ist, R⁶ eine C₁-C₆ Alkylgruppe ist, R⁹ eine C₁-C₆ Alkylgruppe ist, R¹⁷ eine C₁-C₆ Alkyl oder eine C₁-C₆ Alkenylgruppe ist, R¹⁹ eine Aralkyl oder eine Heteroaralkylgruppe ist, R²⁰ eine C₁-C₄ Alkylgruppe ist und m gleich 1 oder 2 ist.

Beispiele für pharmakologisch akzeptable Salze der Verbindungen der Formel (II) sind Salze von physiologisch akzeptablen Mineralsäuren wie Salzsäure, Schwefelsäure und Phosphorsäure oder Salze von organischen Säuren wie Methansulfonsäure, p-Toluolsulfonsäure, Milchsäure, Essigsäure, Ameisensäure, Trifluoressigsäure, Zitronensäure, Bernsteinsäure, Fumarsäure, Maleinsäure und Salicylsäure. Verbindungen der Formel (II) können solvatisiert, insbesondere hydratisiert sein. Die Hydratisierung kann z.B. während des Herstellungsverfahrens oder als Folge der hygroskopischen Natur der anfänglich wasserfreien Verbindungen der Formel (II) auftreten. Wenn die Verbindungen der Formel (II) asymmetrische C-Atome enthalten, können sie entweder als Diastereomeren-Gemische, Gemische von Enantiomeren oder als optisch reine Verbindungen vorliegen.

Die pharmazeutischen Zusammensetzungen gemäß der vorliegenden Erfindung enthalten mindestens eine Verbindung der Formel (II) als Wirkstoff und fakultativ Trägerstoffe und/oder Adjuvantien.

Pro-Drugs bestehen aus einer Verbindung der Formel (II) und mindestens einer pharmakologisch akzeptablen Schutzgruppe, die unter physiologischen Bedingungen abgespalten wird, z.B. einer Alkoxy-, Aralkyloxy-, Acyl- oder Acyloxy-Gruppe, wie z.B. einer Ethoxy-, Benzyloxy-, Acetyl- oder Acetyloxy-Gruppe. Des weiteren beschreibt die vorliegende Erfindung Konjugate, die mindestens eine Verbindung der Formel (II) und einen Antikörper wie z. B. Oligosaccharide, monoklonale Antikörper, Lectine, PSA (Prostata spezifisches Antigen) oder peptidische Vektoren sowie gegebenenfalls einen Linker enthalten. Der Ausdruck Linker bezieht sich auf eine Gruppe, die dazu geeignet ist, Moleküle mit dem Antikörper zu verbinden. Ein Linker kann eine Alkyl-, Heteroalkyl-, Aryl-, Heteroaryl-, Cycloalkyl-, Heterocycloalkyl-, Aralkyl- oder ein Heteroaralkylgruppe sein.

Die therapeutische Verwendung der Verbindungen der Formel (II), ihrer pharmakologisch akzeptablen Salze bzw. Solvate und Hydrate sowie Formulierungen und pharmazeutischen Zusammensetzungen werden ebenfalls beschrieben.

Auch die Verwendung dieser Wirkstoffe zur Herstellung von Arzneimitteln zur Behandlung von Krebserkrankungen ist Gegenstand der vorliegenden Erfindung. Des weiteren sind die vorliegenden Verbindungen bei der Vorbeugung und/oder Behandlung von rheumatoider Arthritis, entzündlichen Erkrankungen, Immunologisch bedingten Krankheiten (z. B. Diabetes Typ 1), Autoimmunkrankheiten, weiteren Tumorerkrankungen sowie zur Oberflächenmodifikation (z. B. Imprägnierung) von Kunststoff- und Metallimplantaten (z. B. Stents) von großem Interesse. Auch sind sie bei der Vorbeugung und/oder Behandlung von Pilzerkrankungen, d.h. als antifugale Mittel einsetzbar. Im allgemeinen werden Verbindungen der Formel (II) unter Anwendung der bekannten und akzeptablen Modi, entweder einzeln oder in Kombination mit einem beliebigen anderen therapeutischen Mittel verabreicht. Solche therapeutisch nützlichen Mittel können auf einem der folgenden Wege verabreicht werden: oral, z.B. als Dragees, überzogene Tabletten, Pillen, Halbfeststoffe, weiche oder harte Kapseln, Lösungen, Emulsionen oder Suspensionen; parenteral, z.B. als injizierbare Lösung; rektal als Suppositorien; durch Inhalation, z.B. als Pulverformulierung oder Spray, transdermal oder intranasal. Zur Herstellung solcher Tabletten, Pillen, Halbfeststoffe, überzogenen Tabletten, Dragees und harten Gelatinekapseln kann das therapeutisch verwendbare Produkt mit pharmakologisch inerten, anorganischen oder organischen Arzneimittelträgersubstanzen vermischt werden, z.B. mit Lactose, Sucrose, Glucose, Gelatine, Malz, Silicagel, Stärke oder Derivaten derselben, Talkum, Stearinsäure oder ihren Salzen, Trockenmagermilch und dgl. Zur Herstellung von weichen Kapseln kann man Arzneimittelträgerstoffe wie z.B. pflanzliche Öle, Petroleum, tierische oder synthetische Öle, Wachs, Fett, Polyole einsetzen. Zur Herstellung von flüssigen Lösungen und Sirups kann man Arzneimittelträgerstoffe wie z.B. Wasser, Alkohole, wäßrige Salzlösung, wäßrige Dextrose, Polyole, Glycerin, pflanzliche Öle, Petroleum, tierische oder synthetische Öle verwenden. Für Suppositorien kann man Arzneimittelträgerstoffe wie z.B. pflanzliche Öle, Petroleum, tierische oder synthetische Öle, Wachs, Fett und Polyole verwenden. Für Aerosol-Formulierungen kann man komprimierte Gase, die für diesen Zweck geeignet sind, wie z.B. Sauerstoff, Stickstoff, Edelgase und Kohlendioxid einsetzen. Die pharmazeutisch verwendbaren Mittel können auch Zusatzstoffe zur Konservierung, Stabilisierung, Emulgatoren, Süßstoffe, Aromastoffe, Salze zur Veränderung des osmotischen Drucks, Puffer, Umhüllungszusatzstoffe und Antioxidantien enthalten.

Kombinationen mit anderen therapeutischen Mitteln können weitere Wirkstoffe beinhalten, die gewöhnlich zur Behandlung von Krebserkrankungen eingesetzt werden.

Zur Synthese von Verbindungen der Formeln (III) werden Verbindungen der Formeln (IV), (V) und (VI) (gegebenenfalls mit gängigen Schutzgruppen versehen) als Bausteine hergestellt. Diese können anschliessend mittels Peptidkupplungen mit an sich üblichen Kupplungsreagenzien wie z. B. Hydroxybenzotriazol (HOBt) und Diisopropylcarbodiimid (DIC) bzw. Dicyclohexylcarbodiimid (DCC) verknüpft werden.

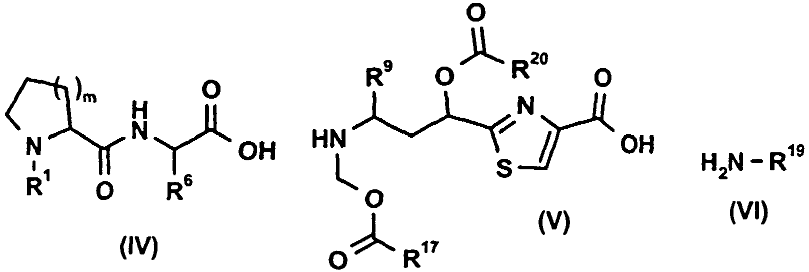

Baustein (IV) kann wiederum durch Peptidkupplung aus kommerziell erhältlichen bzw. in der Literatur beschriebenen Aminosäuren hergestellt werden.

Baustein (V) kann z. B. über eine Multikomponentenreaktion aus Verbindungen der Formeln (VII), (VIII) und (IX) hergestellt werden.

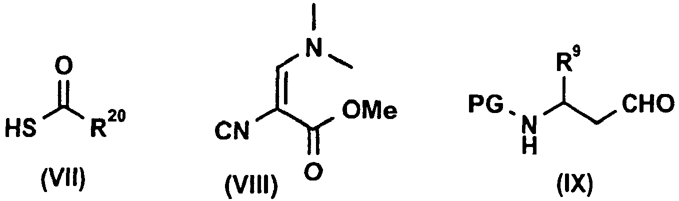

Dabei ist PG eine an sich übliche Aminschutzgruppe wie z. B. tert-Butyloxycarbonyl (Boc). Die dabei hergestellte Verbindung kann mit R¹⁷COOCH₂Cl oder mit H₂CO und R¹⁷ COOH oder mit H₂CO, TMS-Cl und R¹⁷COONa in den Baustein (V) umgewandelt werden (I. Kornonen et al. Acta Chem. Scand. Ser. B 1982, 36(7), 467-474; R. Moriera et al. Tetrahedron Lett. 1994, 35(38), 7107-7110; R. W. A. Luke, Tetrahedron Lett. 1996, 37(2), 263-266).

Bausteine (VI) mit folgender Formel: 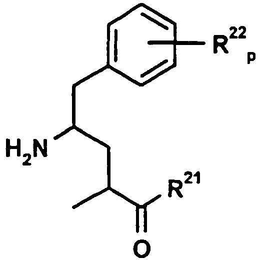 können stereoselektiv über eine Evans-Reaktion hergestellt werden.

Alternativ zu den klassischen Peptidkupplungen können Verbindungen der Formeln (III) auch nach folgendem Schema hergestellt werden:

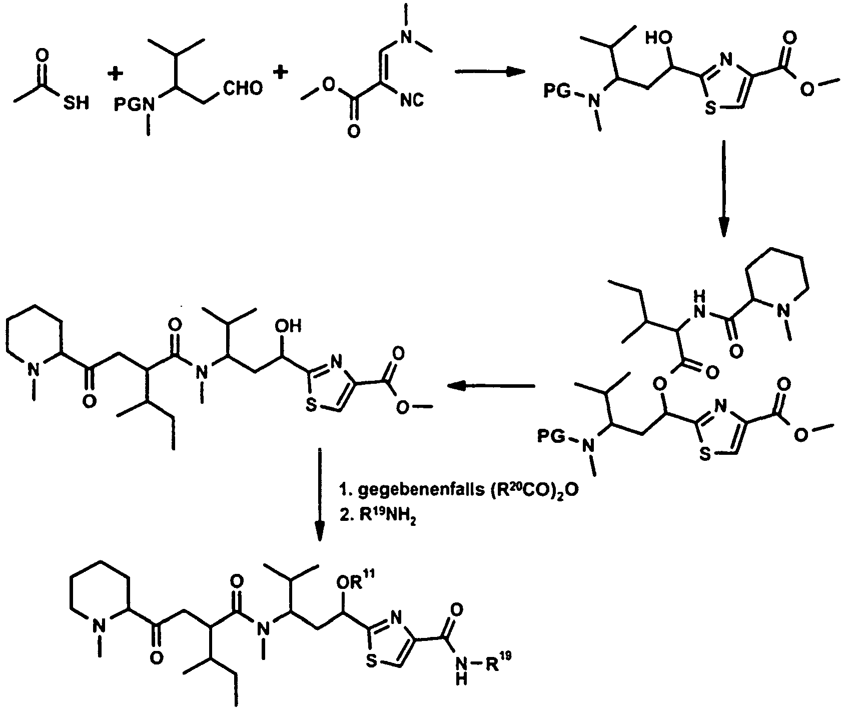

### Beispiele

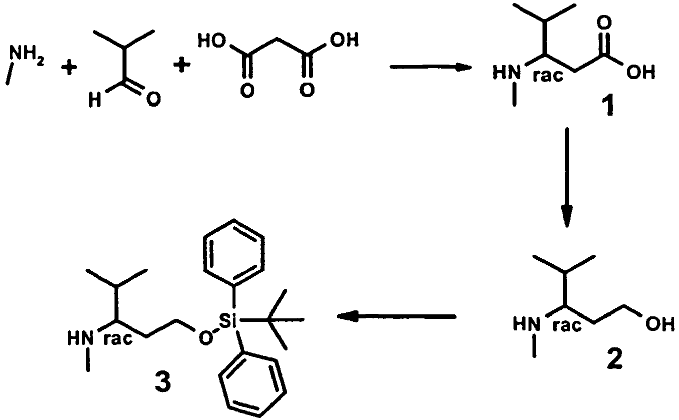

### Synthese von N-Methyl-β-R,S-valin (1)

33.8g Isobutyraldehyd (0.47mol) werden in 200ml Ethanol gelöst. Dann werden 58.8ml (0.47mol) einer 8M Methylamin-Lösung in Ethanol langsam zugetropft unter Eiskühlung. Anschließend werden 50ml THF zugegeben und diese Mischung 1h am Rückfluß erhitzt. Danach wird 48.91g (0.47mol) Malonsäure in kleinen Portionen zugegeben und weitere 5h am Rückfluß erhitzt. Nach dem Abkühlen wird der entstandene Niederschlag abfiltriert, mit THF gewaschen und im Hochvakuum getrocknet. Ausbeute: 50.34g N-Methyl-β-R,S-valin. Massenspektrometrie: Gesuchtes Molekulargewicht 145.2; gefunden: m/z (M+H)⁺ = 146.1.

### Synthese von N-Methyl-β-R,S-valinol (2)

Zu einer Lösung von 150ml 1M Lithiumaluminiumhydrid in THF (0.15mol) werden zunächst 135ml absolutes THF und 14.5g (0.1mol) N-Methyl-β-R,S-valin in kleinen Portionen unter Eiskühlung gegeben. Diese Mischung wird dann 4h am Rückfluß gekocht. Anschließend wird noch über Nacht bei Raumtemperatur gerührt. Danach wird hydrolisiert mit 4ml 12%iger KOH-Lösung und 4ml Wasser. Der entstandene Feststoff wird abfiltriert und zweimal mit je 80ml THF am Rotationsverdampfer ausgekocht. Die Filtrate werden vereint und zur Trockne einrotiert. Das erhaltene Öl wird mittels Destillation fraktioniert (Kp.: 48°C bei 0.5mbar). Ausbeute: 8.28g N-Methyl-β-R,S-valinol. Massen-spektrometrie: Gesuchtes Molekulargewicht 131.2; gefunden: m/z (M+H)⁺ = 132.2.

### Synthese von N-Methyl-β-R,S-valinolyl-tert.-butyldiphenyl-silylether (3)

2g N-Methyl-β-R,S-valinol (15.24mmol) werden in 20ml absolutem Dichlormethan gelöst zusammen mit 465.5mg Dimethylaminopyridin (3.81mmol) und 2.66ml Triethylamin (19.05mmol). Anschließend werden 4.61ml tert.-Butyldiphenylsilylchlorid (18mmol) zugegeben und diese Mischung über Nacht gerührt. Nun werden 20ml Wasser und 20ml Dichlormethan zugegeben und die Phasen getrennt. Die wässrige Phase wird noch zweimal mit Dichlormethan extrahiert und die vereinten organischen Extrakte über Natriumsulfat getrocknet. Das Trockenmittel wird abfiltriert und das Lösungsmittel abgezogen. Der Rückstand wird mittels Säulenchromatographie gereinigt (Eluent: Ethylacetat/Ethanol = 8:2). Ausbeute: 3.94g N-Methyl-β-R,S-valinolyl-tert.butyldiphenylsilylether.
Massenspektrometrie: Gesuchtes Molekulargewicht 369.6; gefunden: m/z (M+H)⁺ = 370.5.

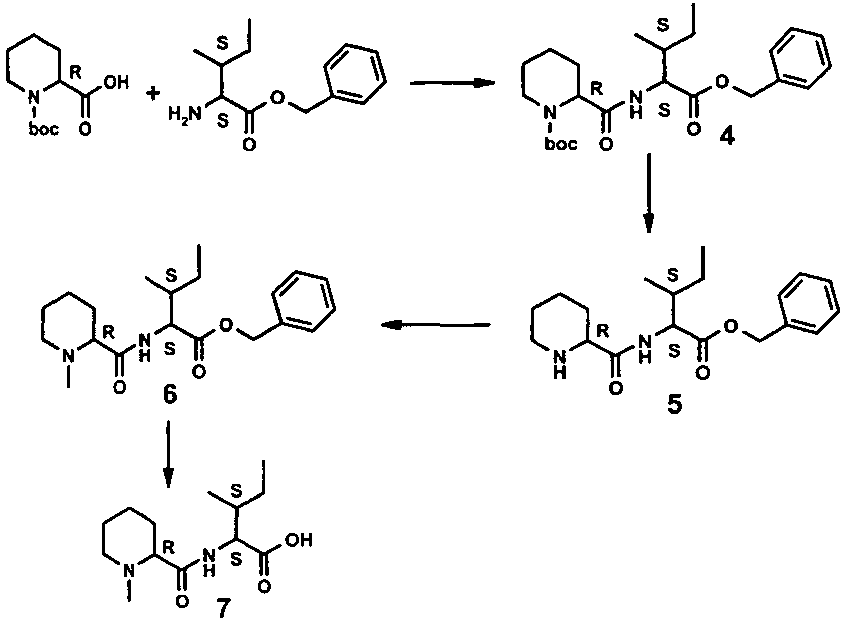

### Darstellung des Dipeptids (R)-N-Boc-HomoPro-(S,S)-Ile-OBzl (4)

Zu einer Lösung von 5g (R)-N-Boc-Homoprolin (21.81mmol) in 40ml trockenem DMF werden 7g 2-(1H-Benzotriazol-1-yl)-1,1,3,3-tetramethyluronium Tetrafluoroborat (TBTU) (21.81mmol) sowie 2.4ml N-Methylmorpholin (21.81mmol) gegeben. Nach 10 Minuten werden 7.21g (S,S)-H-Ile-OBzl Tosylat (18.32mmol) und 2ml N-Methylmorpholin (18.32mmol) zugesetzt. Diese Mischung wird über Nacht bei Raumtemperatur gerührt und dann 40ml Essigester zugegeben. Die organische Phase wird nun mit gesättigter Natriumhydrogencarbonat-Lösung extrahiert. Die wässrige Phase wird noch zweimal mit Essigester extrahiert. Die vereinten organischen Extrakte werden mit gesättigter NaCl-Lösung gewaschen und über Natriumsulfat getrocknet. Zum Schluß wird das Lösungsmittel abgezogen, wobei das Produkt rein anfällt. Ausbeute 5.54g (R)-N-Boc-HomoPro-(S,S)-Ile-OBzl. Massenspektrometrie: Gesuchtes Molekulargewicht 432.6; gefunden: m/z (M+H)⁺ = 433.6.

### Boc-Abspaltung von (R)-N-Boc-HomoPro-(S,S)-Ile-OBzl (5)

(R)-N-Boc-HomoPro-(S,S)-Ile-OBzl wird in 60ml wasserfreiem THF gelöst und unter Eiskühlung 120ml 4M HCl in Dioxan zugesetzt. Man läßt die Mischung auf Raumtemperatur kommen und rührt noch weitere 5h. Das Lösungsmittel wird evaporiert und das erhaltene Rohprodukt direkt weiterverarbeitet. Ausbeute: 4.1g (R)-H-HomoPro-(S,S)-Ile-OBzl. Massenspektrometrie: Gesuchtes Molekulargewicht 332.5; gefunden: m/z (M+H)⁺ = 333.6.

### Reduktive Aminierung von (R)-H-HomoPro-(S,S)-Ile-OBzl (6)

4.1g (R)-HomoPro-(S,S)-Ile-OBzl (12.3mmol) werden in 20ml Methanol gelöst und mit 10ml 37%iger Formalinlösung (123mmol) versetzt. Mit Essigsäure wird pH 5-6 eingestellt und 1.932g Natriumcyanoborhydrid (30.75mmol) portionsweise zugesetzt. Es wird 16h bei Raumtemperatur gerührt und dann die Reaktion mit konz. HCl angesäuert. Das Lösungsmittel wird abgezogen und Wasser zugesetzt. Mit festem NaOH wird pH 12 eingestellt und dreimal mit Dichlormethan extrahiert. Die organische Phase wird über Natriumsulfat getrocknet und anschließend das Lösungsmittel abgezogen. Das resultierende Öl wird mittels Säulenchromatographie gereinigt (Eluent: Ethylacetat : n-Heptan = 1:1). Ausbeute: 3.9g (R)-N-Methyl-HomoPro-(S,S)-Ile-OBzl. Massenspektrometrie: Gesuchtes Molekulargewicht 346.5; gefunden: m/z (M+H)⁺ = 347.4.

### Hydrierung von (R)-N-Methyl-HomoPro-(S,S)-Ile-OBzl (7)

3.9g (R)-N-Methyl-HomoPro-(S,S)-Ile-OBzl (11.26mmol) werden in 30ml Methanol gelöst und 1.2g Pd (10% auf C) zugesetzt. Die Mischung wird zunächst mit Stickstoff gespült und anschließend 10 Minuten Wasserstoff durch die Suspension geleitet. Es wird noch weitere 2h unter Wasserstoff gerührt (Wasserstoffballons) und dann der Katalysator über Celite abfiltriert, welches zweimal mit Methanol nachgewaschen wird. Nach der Evaporation des Lösungsmittels wird ein Öl erhalten, das nach Lyophilisation ein weißes Pulver ergibt. Ausbeute: 2.7g (R)-N-Methyl-HomoPro-(S,S)-Ile-OH. Massenspektrometrie: Gesuchtes Molekulargewicht 256.4; gefunden: m/z (M+H)⁺ = 257.4.

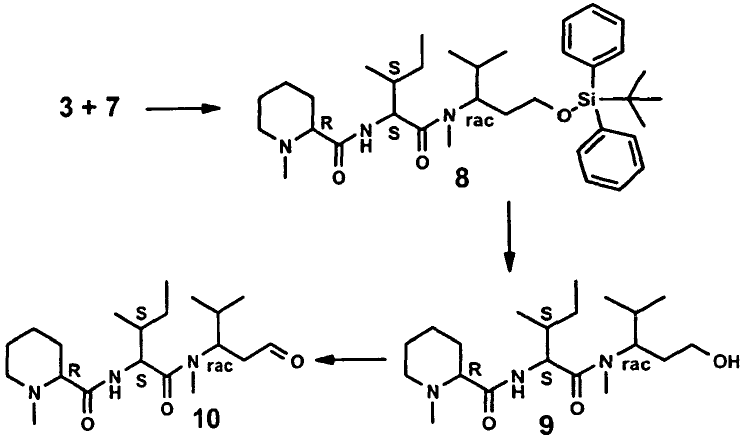

### Kupplung von (R)-N-Methyl-HomoPro-(S,S)-Ile-OH mit N-Methyl-β-R,S-valinolyl-tert.butyldiphenylsilylether (8)

3.522g (R)-N-Methyl-HomoPro-(S,S)-Ile-OH (13.74mmol) werden in 15ml absolutem DMF gelöst und 2.104g Hydoxybenzotriazol (13.74mmol) sowie 2.151ml Diisopropylcarbodiimid (13.74mmol) zugesetzt. Nach 15minütigem Rühren werden 4.232g N-Methyl-β-R,S-valinolyl-tert.butyldiphenylsilylether (11.45mmol) zugegeben und die Mischung 16h bei Raumtemperatur gerührt. Der ausgefallene Diisopropylharnstoff wird abfiltriert und dann die Lösung zur Trockne einrotiert. Rückstand wird in Dichlormethan aufgenommen und restlicher Harnstoff abfiltriert. Die Dichlormethan-Lösung wird mit gesättigter Natriumhydrogencarbonat-Lösung ausgeschüttelt und anschließend über Natriumsulfat getrocknet. Das Trockenmittel wird abgetrennt und und das Lösungsmittel abgezogen. Der Rückstand wird mittels präparativer HPLC gereinigt (Reversed Phase-C18-Phase, Eluent Methanol+0.5%Essigsäure / Wasser+0.5% Essigsäure). Ausbeute: 3.91g. Massenspektrometrie: Gesuchtes Molekulargewicht 608.0; gefunden: m/z (M+H)⁺ = 609.0.

### Abspaltung der tert.Butyldiphenylsilyl-Schutzgruppe von (8) (9)

3.91g (8) (6.43mmol) werden in 30ml Tetrahydrofuran abs. gelöst. Dann werden tropfenweise 2.223ml Tetrabutylammoniumfluorid-Lösung (1M in THF) (7.72mmol) zugegeben und 2h bei Raumtemperatur gerührt. Danach wird mit 8ml Wasser hydrolisiert und Tetrahydrofuran abrotiert. Die Lösung wird neutralisiert und fünfmal mit Ethylacetat extrahiert. Die vereinten organischen Extrakte werden noch zweimal mit gesättigter NaCl-Lösung ausgeschüttelt und über Natriumsulfat getrocknet. Nach dem Abtrennen des Trockenmittels wird zur Trockne einrotiert. Das Rohprodukt wird dann direkt weiterverarbeitet. Ausbeute wurde nicht bestimmt, da sich noch Diphenyltert.butylsilanol im Gemisch befindet. Massenspektrometrie: Gesuchtes Molekulargewicht 369.6; gefunden: m/z (M+H)⁺ = 370.5.

### Swern-Oxidation von (9) zu (10)

0.665ml Oxalylchlorid (7.75mmol) werden einem 250ml-Kolben unter Stickstoff in 25ml absolutem Dichlormethan gelöst und auf -70°C runtergekühlt. Dann werden langsam 1.188ml Dimethylsulfoxid (16.73mmol) in 5ml Dichlormethan zugetropft (Temperatur nicht über -60°C) und noch weitere 30 Minuten bei tiefer Temperatur gerührt. Anschließend wird eine Lösung (6ml) von (9) (6.43mmol) in Dichlormethan zugetropft (Temperatur nicht über -60°C). Es wird nochmals 30 Minuten gerührt und bei tiefer Temperatur 4.459ml Triethylamin (32.17mmol) zugegeben. Sobald die Mischung Raumtemperatur erreicht hat, werden 15ml Wasser zugegeben und noch weitere 10 Minuten gerührt. Die Phasen werden getrennt und die wässrige Phase noch zweimal mit Dichlormethan extrahiert. Nach dem Trocknen der organischen Phase über Natriumsulfat wird zur Trockne einrotiert. Das erhaltene Rohprodukt wurde im nächsten Schritt weiterverarbeitet, Ausbeute konnte deshalb nicht bestimmt werden. Massenspektrometrie: Gesuchtes Molekulargewicht 367.6; gefunden: m/z (M+H)⁺ = 368.5.

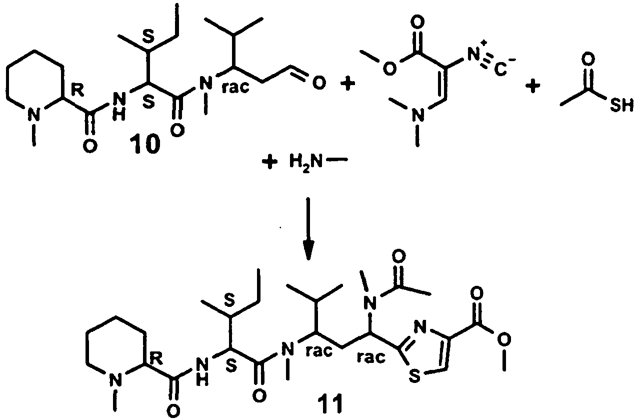

### Thiazolsynthese (11)

0.695ml Methylamin-Lösung (33% in Ethanol) (7.72mmol) werden zu (10) in 20ml absolutem Methanol gegeben und 1h bei Raumtemperatur gerührt. Anschließend werden 991.3mg 3-Dimethylamino-2-isocyano-acrylsäuremethylester (6.43mmol) und 0.457ml Thioessigsäure (6.43mmol) zugegeben und 16h bei Raumtemperatur gerührt. Das Lösungsmittel wird dann abgezogen und der Rückstand mittels präparativer HPLC gereinigt (Reversed Phase-C18-Phase, Eluent Methanol+0.5%Essigsäure / Wasser+0.5% Essigsäure). Ausbeute: 1.294g. Massenspektrometrie: Gesuchtes Molekulargewicht 565.8; gefunden: m/z (M+H)⁺ = 566.7.

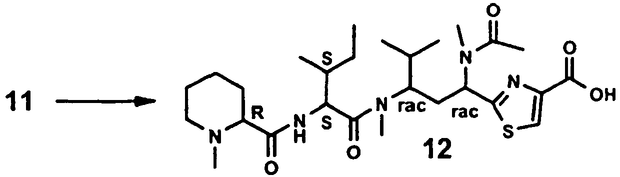

### Verseifung von (11) zu (12)

1.294g (11) (2.29mmol) werden in 20ml Tetrahydrofuran gelöst und 220mg LiOH (9.16mmol) in 20ml Wasser zugegeben. Diese Mischung wird 16h bei Raumtemperatur gerührt und dann mit 2N HCl neutralisiert. Anschließend wird das Lösungsmittel abgezogen und der Rückstand mittels präparativer HPLC von LiCl befreit (Reversed Phase-C18-Phase, Eluent Methanol+0.5%Essigsäure / Wasser+0.5% Essigsäure). Ausbeute: 1.14g. Massenspektrometrie: Gesuchtes Molekulargewicht 551.8; gefunden: m/z (M+H) ⁺ = 552.7.

### Referenzbeispiele 13 - 34:

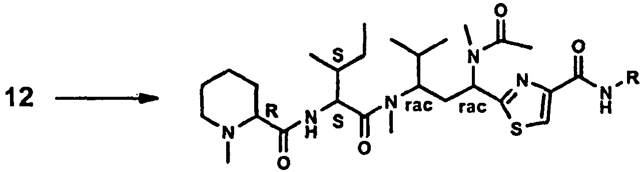

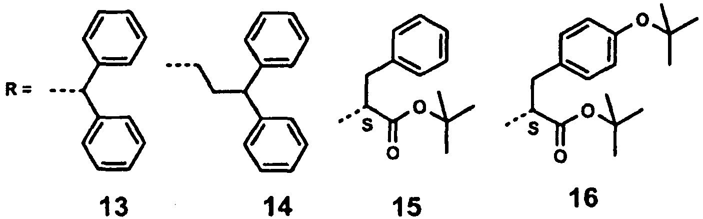

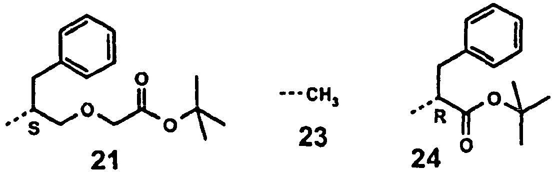

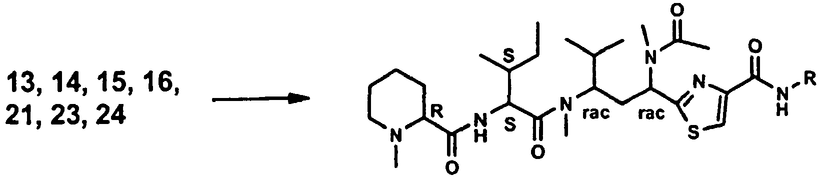

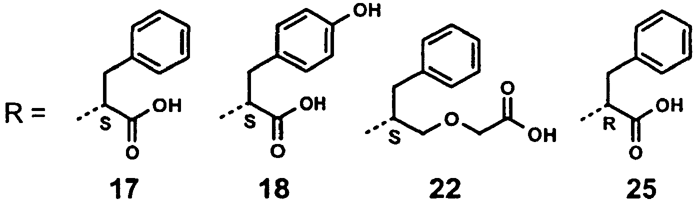

### Kupplung von (12) mit α-Aminodiphenylmethan (13)

49.5mg (12) (0.09mmol) werden in 3ml absolutem DMF gelöst und 18.6mg 6-Chlorhydroxybenzotriazol (0.11mmol) sowie 0.014ml Diisopropylcarbodiimid (0.11mmol zugegeben. Diese Mischung wird 15 Minuten bei Raumtemperatur gerührt und dann 0.062ml α-Aminodiphenylmethan (0.36mmol) zugesetzt. Es wird über Nacht bei Raumtemperatur gerührt, die Lösung dann filtriert und der Rückstand mittels präparativer HPLC gereinigt (Reversed Phase-C18-Phase, Eluent Methanol+0.5%Essigsäure / Wasser+0.5% Essigsäure). Ausbeute: 35mg. Massenspektrometrie: Gesuchtes Molekulargewicht 717.0; gefunden: m/z (M+H)⁺ = 718.1.

### Kupplung von (12) mit 3,3-Diphenylpropylamin (14)

49.5mg (12) (0.09mmol) werden in 3ml absolutem DMF gelöst und 18.6mg 6-Chlorhydroxybenzotriazol (0.11mmol) sowie 0.014ml Diisopropylcarbodiimid (0.11mmol) zugegeben. Diese Mischung wird 15 Minuten bei Raumtemperatur gerührt und dann 76mg 3,3-Diphenylpropylamin (0.36mmol) zugesetzt. Es wird über Nacht bei Raumtemperatur gerührt, die Lösung dann filtriert und der Rückstand mittels präparativer HPLC gereinigt (Reversed Phase-C18-Phase, Eluent Methanol+0.5%Essigsäure / Wasser+0.5% Essigsäure). Ausbeute: 31mg. Massenspektrometrie: Gesuchtes Molekulargewicht 745.0; gefunden: m/z (M+H)⁺ = 746.1.

### Kupplung von (12) mit S-Phenylalanintert.butylester (15)

49.5mg (12) (0.09mmol) werden in 3ml absolutem DMF gelöst und 18.6mg 6-Chlorhydroxybenzotriazol (0.11mmol) sowie 0.014ml Diisopropylcarbodiimid (0.11mmol) zugegeben. Diese Mischung wird 15 Minuten bei Raumtemperatur gerührt und dann 24.3mg S-Phenylalanintert.butylester (0.11mmol) zugesetzt. Es wird über Nacht bei Raumtemperatur gerührt, die Lösung dann filtriert und der Rückstand mittels präparativer HPLC gereinigt (Reversed Phase-C18-Phase, Eluent Methanol+0.5%Essigsäure / Wasser+.0.5% Essigsäure). Ausbeute: 26mg. Massenspektrometrie: Gesuchtes Molekulargewicht 755.0; gefunden: m/z (M+H)⁺ = 756.2.

### Kupplung von (12) mit S-Tyrosin-O-tert.-butylether-tert.-butylester (16)

49.5mg (12) (0.09mmol) werden in 3ml absolutem DMF gelöst und 18.6mg 6-Chlorhydroxybenzotriazol (0.11mmol) sowie 0.014ml Diisopropylcarbodiimid (0.11mmol) zugegeben. Diese Mischung wird 15 Minuten bei Raumtemperatur gerührt und dann 32.3mg S-Tyrosin-O-tert.-butylether-tert.-butylester (0.11mmol) zugesetzt. Es wird über Nacht bei Raumtemperatur gerührt, die Lösung dann filtriert und der Rückstand mittels präparativer HPLC gereinigt (Reversed Phase-C18-Phase, Eluent Methanol+0.5%Essigsäure / Wasser+0.5% Essigsäure). Ausbeute: 28mg. Massen-spektrometrie: Gesuchtes Molekulargewicht 827.1; gefunden: m/z (M+H)⁺ = 828.0.

### Entschützung von (15) zu (17)

26mg (15) (0.034mmol) werden in 2ml absolutem Dichlormethan gelöst und dann 2ml Trifluoressigsäure zugegeben. Diese Mischung wird 1h bei Raumtemperatur gerührt und dann unter Zusatz von n-Heptan zur Trockne einrotiert. Produkt fällt rein an. Ausbeute: 20mg. Massenspektrometrie: Gesuchtes Molekulargewicht 698.9; gefunden: m/z (M+H)⁺ = 699.5.

### Entschützung von (16) zu (18)

28mg (16) (0.034mmol) werden in 2ml absolutem Dichlormethan gelöst und dann 2ml Trifluoressigsäure zugegeben. Diese Mischung wird 1h bei Raumtemperatur gerührt und dann unter Zusatz von n-Heptan zur Trockne einrotiert. Produkt fällt rein an. Ausbeute: 18mg. Massenspektrometrie: Gesuchtes Molekulargewicht 714.9; gefunden: m/z (M+H)⁺ = 715.6.

### Kupplung von Benzyloxycarbonyl-S-phenylalaninol mit Bromessigsäure-tert.-butyl-ester (19)

1.141g Benzyloxycarbonyl-S-phenylalaninol (4mmol) werden mit 160mg Natriumhydrid-Dispersion (60%ig in Mineralöl) in 20ml absolutem THF umgesetzt. Nach beender Wasserstoffentwicklung werden 1.182ml Bromessigsäure-tert.-butylester (8mmol) zugegeben und 48h bei Raumtemperatur gerührt. Die Lösung wird zur Trockne einrotiert und das Produkt mittels präparativer HPLC gereinigt. (Reversed Phase-C18-Phase, Eluent Methanol+0.5%Essigsäure / Wasser +0.5% Essigsäure). Ausbeute: 805mg.
Massenspektrometrie: Gesuchtes Molekulargewicht 399.5; gefunden: m/z (M+H)⁺ = 400.3.

### Abspaltung der Benzyloxycarbonyl-Schutzgruppe von (19) (20)

805mg (19) (2.02mmol) werden in 15ml Methanol unter Inertgas gelöst und 800mg Palladium auf Aktivkohle (10%) zugestzt. Kolben wird mit einem Septum verschlossen und mit zwei Wasserstoffballons verknüpft. Es wird 16h bei Raumtemperatur gerührt und danach der Katalysator über Celite abfiltriert und mehrmals mit Methanol nachgewaschen. Zum Schluß wird das Lösungsmittel abgezogen. Ausbeute: 482mg. Massenspektrometrie: Gesuchtes Molekulargewicht 265.4; gefunden: m/z (M+H)⁺ = 266.3.

### Kupplung von (12) mit (20) (21)

49.5mg (12) (0.09mmol) werden in 3ml absolutem DMF gelöst und 16.8mg Hydroxybenzotriazol Hydrat (0.11mmol) sowie 0.014ml Diisopropylcarbodiimid (0.11mmol) zugegeben. Diese Mischung wird 15 Minuten bei Raumtemperatur gerührt und dann 29.2mg (20) (0.11mmol) zugesetzt. Es wird über Nacht bei Raumtemperatur gerührt, die Lösung dann filtriert und der Rückstand mittels präparativer HPLC gereinigt (Reversed Phase-C18-Phase, Eluent Methanol+0.5%Essigsäure / Wasser+0.5% Essigsäure). Ausbeute: 22mg. Massenspektrometrie: Gesuchtes Molekulargewicht 799.1; gefunden: m/z (M+H)⁺ = 800.2.

### Entschützung von (21) zu (22)

22mg (21) (0.028mmol) werden in 2ml absolutem Dichlormethan gelöst und dann 2ml Trifluoressigsäure zugegeben. Diese Mischung wird 1h bei Raumtemperatur gerührt und dann unter Zusatz von n-Heptan zur Trockne einrotiert. Produkt fällt rein an. Ausbeute: 16mg. Massenspektrometrie: Gesuchtes Molekulargewicht 757.0; gefunden: m/z (M+H)⁺ = 758.2.

### Kupplung von (12) mit Methylamin (23)

49.5mg (12) (0.09mmol) werden in 3ml absolutem DMF gelöst und 18.6mg 6-Chlorhydroxybenzotriazol (0.11mmol sowie 0.014ml Diisopropylcarbodiimid (0.11mmol) zugegeben. Diese Mischung wird 15 Minuten bei Raumtemperatur gerührt und dann 0.22ml Methylamin-Lösung (2M in THF) (0.44mmol) zugesetzt. Es wird über Nacht bei Raumtemperatur gerührt, die Lösung dann filtriert und der Rückstand mittels präparativer HPLC gereinigt (Reversed Phase-C18-Phase, Eluent Methanol+0.5%Essigsäure / Wasser+0.5% Essigsäure). Ausbeute: 14mg. Massenspektrometrie: Gesuchtes Molekulargewicht 564.8; gefunden: m/z (M+H)⁺ = 565.7.

### Kupplung von (12) mit R-Phenylalanintert.butylester (24)

49.5mg (12) (0.09mmol) werden in 3ml absolutem DMF gelöst und 16.8mg Hydroxybenzotriazol (0.11mmol) sowie 0.014ml Diisopropylcarbodiimid (0.11mmol) zugegeben. Diese Mischung wird 15 Minuten bei Raumtemperatur gerührt und dann 24.3mg R-Phenylalanintert.butylester (0.11mmol) zugesetzt. Es wird über Nacht bei Raumtemperatur gerührt, die Lösung dann filtriert und der Rückstand mittels präparativer HPLC gereinigt (Reversed Phase-C18-Phase, Eluent Methanol+0.5%Essigsäure / Wasser+0.5% Essigsäure). Ausbeute: 23mg. Massenspektrometrie: Gesuchtes Molekulargewicht 755.0; gefunden: m/z (M+H)⁺ = 756.2.

### Entschützung von (24) zu (25)

23mg (24) (0.03mmol) werden in 2ml absolutem Dichlormethan gelöst und dann 2ml Trifluoressigsäure zugegeben. Diese Mischung wird 1h bei Raumtemperatur gerührt und dann unter Zusatz von n-Heptan zur Trockne einrotiert. Produkt fällt rein an. Ausbeute: 18mg. Massenspektrometrie: Gesuchtes Molekulargewicht 698.9; gefunden: m/z (M+H)⁺ = 699.5.

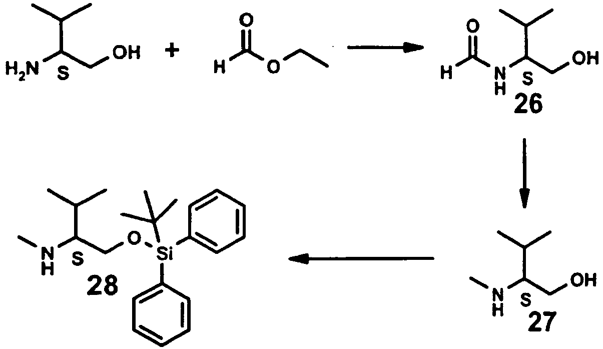

### Synthese von N-Formyl-S-valinol (26)

10g S-Valinol (97mmol) werden in 50ml Ethylformiat gelöst und 1h am Rückfluß gekocht. Das Lösungsmittel wird abrotiert und der Rückstand im Vakuum destilliert (Kp.: 153°C bei 0.5mbar). Ausbeute: 8.4g. Massenspektrometrie: Gesuchtes Molekulargewicht 131.2; gefunden: m/z (M+H)⁺ = 132.3.

### Synthese von N-Methyl-S-valinol (27)

8.4g N-Formyl-S-valinol (64mmol) werden in 40 ml absolutem Tetrahydrofuran gelöst und tropfenweise zu einem Gemisch aus 5.7g Lithiumaluminiumhydrid (150mmol) in 200ml absolutem Tetrahydrofuran gegeben. Diese Mischung wird nun 16h bei Raumtemperatur gerührt. Anschließend werden portionsweise 30g Natriumsulfat Decahydrat und 18ml Wasser zugesetzt und für weitere 3h bei Raumtemperatur gerührt. Der Feststoff wird nun abfiltriert und das Filtrat zur Trockne einrotiert. Der resultierende Rückstand wird im Vakuum fraktioniert (Kp.: 93°C bei 54mbar). Ausbeute: 3.7g. Massenspektrometrie: Gesuchtes Molekulargewicht 117.2 gefunden: m/z (M+H)⁺ = 118.1.

### Synthese von N-Methyl-S-valinolyl-tert.butyldiphenylether (28)

1.64g N-Methyl-S-valinol (14mmol) werden in 10ml absolutem Dichlormethan gelöst und 427mg Dimethylaminopyridin (3.5mmol) sowie 2.44ml Triethylamin (17.5mmol) zugegeben. Anschließend werden 4.3ml tert.Butyldiphenylsilylchlorid zugesetzt und 16h bei Raumtemperatur gerührt. Dann werden jeweils 10ml Wasser und Tetrahydrofuran zur Mischung gegeben und die Phasen getrennt. Die wässrige Phase wird noch zweimal mit Dichlormethan extrahiert. Die vereinten organischen Extrakte werden über Natriumsulfat getrocknet und danach die Lösung zur Trockne einrotiert. Der Rückstand wird mittels Säulenchromatographie gereinigt (Eluent: Ethylacetat/Ethanol = 8:2). Ausbeute: 3.16g. Massen-spektrometrie: Gesuchtes Molekulargewicht 355.6 gefunden: m/z (M+H)⁺ = 366.6.

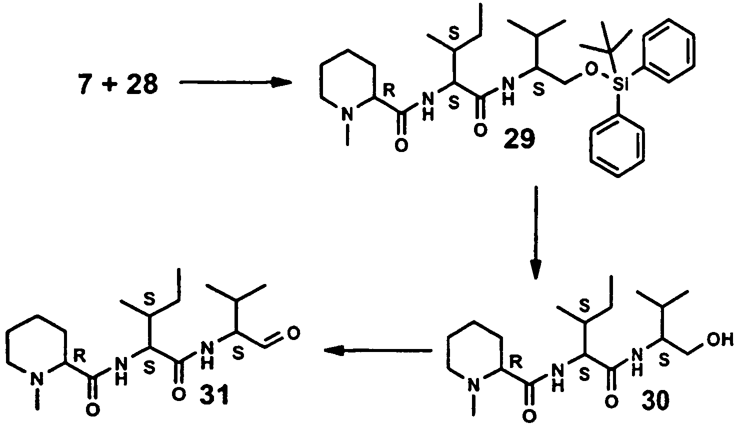

### Kupplung von (R)-N-Methyl-HomoPro-(S,S)-Ile-OH mit NMethyl-S-valinolyl-tert.butyldiphenylsilylether (29)

1.54g (R)-N-Methyl-HomoPro-(S,S)-Ile-OH (6mmol) werden in 10ml absolutem DMF gelöst und 1.02g 6-Chlorohydroxybenzotriazol (6mmol) sowie 0.939ml Diisopropylcarbodiimid (6mmol) zugesetzt. Diese Mischung wird 15 Minuten gerührt und dann 2.56g N-Methyl-*S-*valinolyl-tert.butyldiphenylether (7.2mmol) zugegeben. Es wird 16h bei Raumtemperatur gerührt. Anschließend wird das Lösungsmittel evaporiert und der Rückstand mittels präparativer HPLC getrennt (Reversed Phase-C18-Phase, Eluent Methanol+0.5%Essigsäure / Wasser+0.5% Essigsäure). Ausbeute: 1.06g. Massenspektrometrie: Gesuchtes Molekulargewicht 593.9 gefunden: m/z (M+H)⁺ = 594.8.

### Abspaltung der tert.-Butyldiphenylsilyl-Schutzgruppe von (29) zu (30)

1.06g (29) (1.79mmol) werden in 10ml absolutem Tetrahydrofuran gelöst und 2.15ml Tetrabutylammoniumfluorid-Lösung (1M Lösung in Tetrahydrofuran) (2.15mmol) zugetropft. Es wird 16h bei Raumtemperatur gerührt und anschließend mit 3ml Wasser hydrolisiert. Tetrahydrofuran wird abrotiert und der Rückstand fünfmal mit Ethylacetat ausgeschüttelt. Die vereinten organischen Extrakte werden mit NaCl-Lösung ausgeschüttelt und dann über Natriumsulfat getrocknet. Das Trockenmittel wird abfiltriert und das Lösungsmittel evaporiert. Rohausbeute: 1.05g (enthält noch abgespaltene Silylschutzgruppe). Massenspektrometrie: Gesuchtes Molekulargewicht 355.5 gefunden: m/z (M+H)⁺ = 356.5.

### Swern-Oxidation von (30) zu (31)

In einem 100ml-Kolben unter Stickstoff werden 0.316ml Oxalylchlorid (1.98mmol) in 3ml wasserfreiem Dichlormethan vorgelegt und auf -70°C abgekühlt. Man tropft dann langsam eine Mischung von 0.305ml Dimethylsulfoxid (4.29mmol) in 0.6ml Dichlormethan zu (Gasentwicklung, Temperatur nicht über -60°C) und rührt dann noch 30 Minuten. Dann tropft man eine Lösung von 587mg (30) (1.65mmol) in 2ml Dichlormethan zu (Temperatur nicht über -60°C). Es wird nochmal 30 Minuten gerührt und dann bei tiefer Temperatur 1.146ml Triethylamin (8.25mmol) zugegeben. Dann läßt man die Mischung zur Raumtemperatur kommen und tropft 10ml Wasser zu und läßt dann noch 10 Minuten rühren. Die Phasen werden dann getrennt und die wässrige Phase noch zweimal mit Dichlormethan ausgeschüttelt. Die vereinten organischen Phasen werden über Natriumsulfat getrocknet. Das Trockenmittel wird abfiltriert und die resultierende Lösung zur Trockne einrotiert. Ausbeute: 636mg Rohprodukt. Massenspektrometrie: Gesuchtes Molekulargewicht 353.5 gefunden: m/z (M+H)⁺ = 354.5.

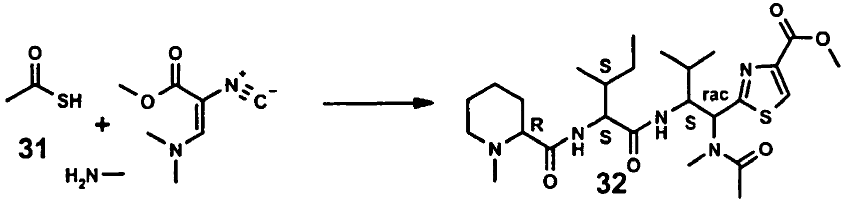

### Thiazolsynthese (32)

636mg (31) (1.15mmol) werden mit 0.173ml Methylamin-Lösung (33% in Ethanol) (1.38mmol) in 3ml absolutem Methanol 1h bei Raumtemperatur gerührt. Anschließend werden 185mg 3-Dimethylamino-2-isocyanoacrylsäuremethylester (1.2mmol) und 0.086ml Thioessigsäure (1.2mmol) zugegeben und 16h bei Raumtemperatur gerührt. Das Lösungsmittel wird dann abgezogen und der Rückstand mittels präparativer HPLC gereinigt (Reversed Phase-C18-Phase, Eluent Methanol+0.5%Essigsäure / Wasser+0.5% Essigsäure). Ausbeute: 150mg. Massenspektrometrie: Gesuchtes Molekulargewicht 551.8; gefunden: m/z (M+H)⁺ = 552.7.

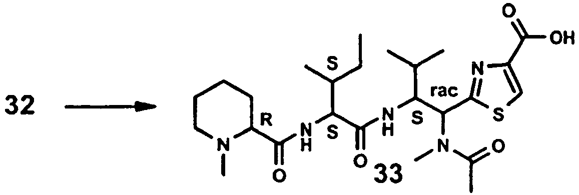

### Verseifung von (32) zu (33)

61g (32) (0.11mmol) werden in 2ml Tetrahydrofuran gelöst und 10.6mg LiOH (0.44mmol) in 2ml Wasser zugegeben. Diese Mischung wird 16h bei Raumtemperatur gerührt und dann mit 2N HCl neutralisiert. Anschließend wird das Lösungsmittel abgezogen und der Rückstand mittels präparativer HPLC von LiCl befreit (Reversed Phase-C18-Phase, Eluent Methanol+0.5%Essigsäure / Wasser+0.5% Essigsäure).
Ausbeute: 50mg. Massenspektrometrie: Gesuchtes Molekulargewicht 537.7; gefunden: m/z (M+H)⁺ = 538.7.

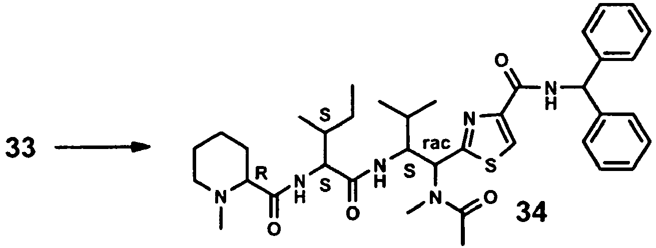

### Kupplung von (33) mit α-Aminodiphenylmethan (34)

49.5mg (33) (0.093mmol) werden in 3ml absolutem DMF gelöst und 14.2mg Hydroxybenzotriazol (0.093mmol) sowie 0.012ml Diisopropylcarbodiimid (0.093mmol) zugegeben. Diese Mischung wird 15 Minuten bei Raumtemperatur gerührt und dann 0.064ml α-Aminodiphenylmethan (0.372mmol) zugesetzt. Es wird über Nacht bei Raumtemperatur gerührt, die Lösung dann filtriert und der Rückstand mittels präparativer HPLC gereinigt (Reversed Phase-C18-Phase, Eluent Methanol +0.5%Essigsäure / Wasser+0.5% Essigsäure).
Ausbeute: 30mg. Massenspektrometrie: Gesuchtes Molekulargewicht 703.0; gefunden: m/z (M+H)⁺ = 704.1.

### Allgemeine Arbeitsvorschrift zur Synthese von Thiazolen:

1 mmol der Carbonylverbinung (IX) wird unter einer Stickstoffatmosphäre in 3 ml abs. THF gelöst und 1 mmol Bortrifluoridetherat zugegeben. Diese Mischung wird nach 10 min mit 1 mmol des Isonitrils (VIII) und 1 mmol der Thiocarbonsäure (VII) versetzt und die Mischung 72h gerührt. Zur Aufarbeitung wird Wasser zugegeben und gegebenenfalls über Celite abfiltriert. Dann wird zur Trockne einrotiert, in Ethylacetat aufgenommen und zweimal mit Wasser ausgeschüttelt. Nach dem Trocknen der organischen Phase über Natriumsulfat wird zur Trockne einrotiert. Der Rückstand wird mittels präparativer HPLC gereinigt (Reversed Phase-C18-Phase, Eluent Methanol+0.5%Essigsäure / Wasser+0.5% Essigsäure).

Verbindungen der Formel (IX) können z. B. über eine α-Aminoalkylierung aus Isobutyraldehyd, Ammoniumacetat oder einem primären Amin bzw. dessen Hydrochlorid und Malonsäure hergestellt werden:

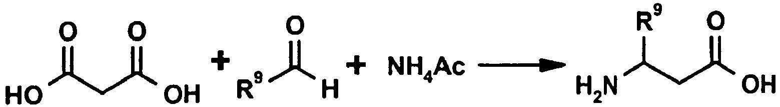

Die dabei entstehende β-Aminosäure wird anschliessend gegebenenfalls N-alkyliert (z. B. durch Reduktive Aminierung) und anschliessend mit einer Schutzgruppe (z. B. t-Butyloxycarbonyl, Boc) versehen. Danach wird die Säuregruppe nach Standartvorschriften (z. B. durch Reduktion zum Alkohol mit LiAlH₄ und anschliessender Swern Oxidation zum Aldehyd) in den Aldehyd überführt (siehe z. B. R. C. Larock, Comprehensive Organic Transformations, VCH Publishers, New York, 1989). Alternativ kann die β-Aminosäure auch über eine Arndt-Eistert-Reaktion aus Valin hergestellt werden.

### Beispiel 35:

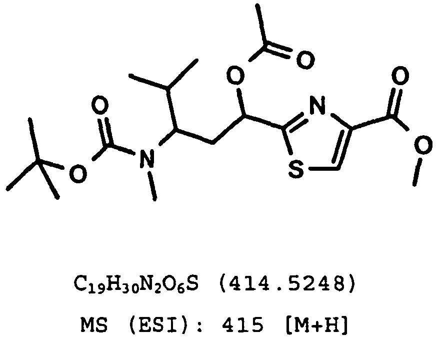

### Beispiel 36:

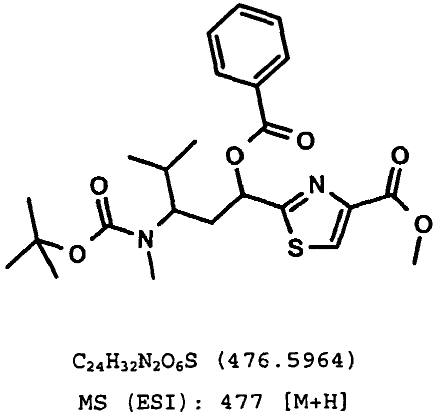

### Beispiel 37:

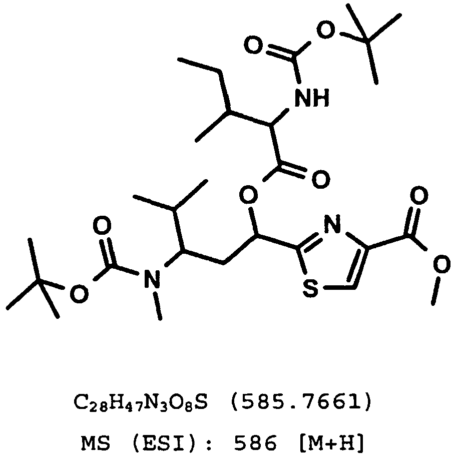

### Beispiel 38:

Die Verbindung aus Beispiel 35 (0.1 mmol) wird in 2 ml Dichlormethan (DCM) und 0.1 ml Trifluoressigsäure (TFA) gelöst und 1 h bei Raumtemperatur gerührt. Anschliessend wird das DCM/TFA-Gemisch im Vakuum entfernt und der Rückstand mittels HPLC gereinigt.

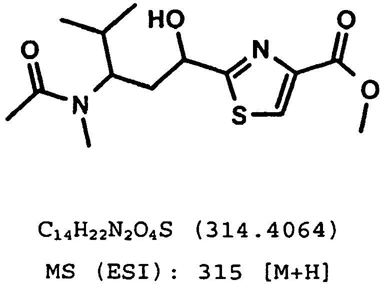

### Beispiel 39:

Die Verbindung aus Beispiel 37 (0.1 mmol) wird in 2 ml Dichlormethan (DCM) und 0.1 ml Trifluoressigsäure (TFA) gelöst und 1 h bei Raumtemperatur gerührt. Anschliessend wird das DCM/TFA-Gemisch im Vakuum entfernt und der Rückstand mittels HPLC gereinigt.

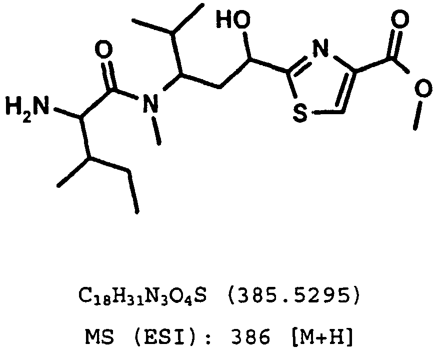

### Beispiel 40:

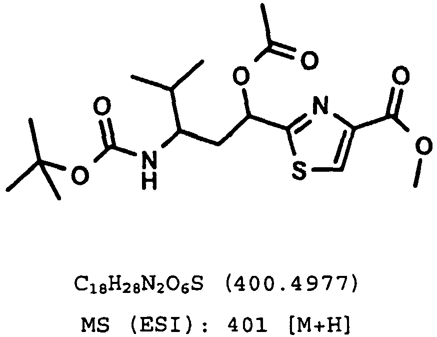

### Beispiel 41:

1 mmol der Verbindung aus Beispiel 40 wird in 1 ml Methanol aufgenommen und mit 1 ml einer 4 M Ammoniak Lösung in Methanol versetzt. Nach 2h rühren bei Raumtemperatur wird das Lösungsmittel im Vakuum entfernt.

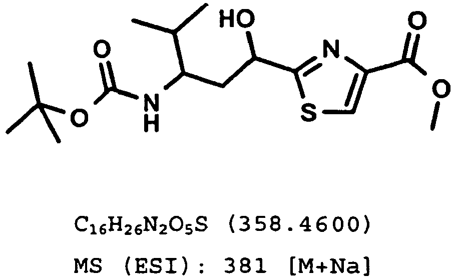

### Beispiel 42 und 43:

### Esterkupplung des Hydroxythiazols (Beispiel 41) mit Dipeptid (7) und anschließender Acylwanderung:

2 mmol (512 mg) 3-Methyl-2-[(1-methyl-piperidin-2carbonyl)-amino]-pentansäure (7) werden in einem Schlenkkolben vorgelegt, evaporiert und mit Stickstoff geflutet. Die Carbonsäure wird in 5 ml trockenem Dichlormethan suspendiert und im Eisbad auf 0°C gekühlt. 2 mmol (252 mg) *N,N'*-Diisopropylcarbodiimid (DIC) und 0,2 mmol (24 mg) DMAP werden in je 2,5 ml DCM gelöst und zugetropft. Man lässt die Mischung 5 min bei 0°C rühren. 1 mmol (372 mg) 2-[3-(tert.-Butoxycarbonyl-methyl-amino)-1hydroxy-4-methyl-pentyl]-thiazol-4-carbonsäuremethylester (Beispiel 41) werden in 5 ml DCM gelöst und langsam mit einer Spritze zugetropft. Man lässt die Reaktionsmischung auf RT erwärmen und weitere 4 h rühren. Die Reaktionslösung wird im Vakuum etwas eingeengt und der ausfallende Harnstoff abfiltriert. Das Filtrat wird mit 1 ml Trifluoressigsäure versetzt und 1 h bei RT gerührt, anschließend wird der Ansatz komplett einrotiert. Der Rückstand wird in 1 ml Dichlormethan aufgenommen, mit 1 ml Triethylamin versetzt und 1 h bei RT gerührt. Das Lösungsmittel wird vollständig abgezogen. Das umgelagerte Kupplungsprodukt wird mittels HPLC gereinigt.

### 2-(3-(tert.-Butoxycarbonyl-methyl-amino)-4-methyl-1-{3methyl-2-[(1-methyl-piperidin-2-carbonyl)-amino]pentanoyloxy}-pentyl)-thiazol-4-carbonsäure-methylester (42) :

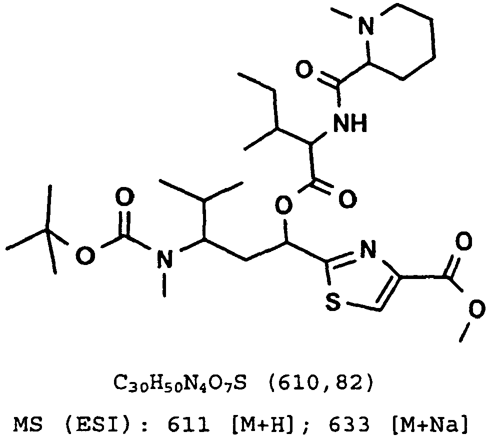

### 2-[1-Hydroxy-4-methyl-3-(methyl-{3-methyl-2-[(1-methylpiperidin-2-carbonyl)-amino]-pentanoyl}-amino)-pentyl]thiazol-4-carbonsäuremethylester (43):

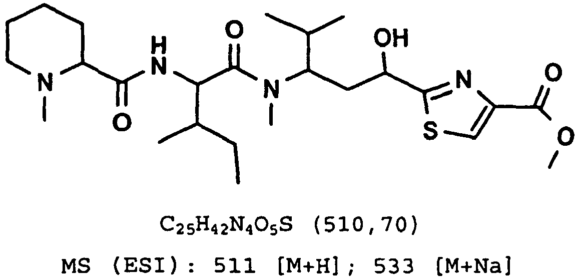

### Beispiel 44 und 45: '

### Umsetzung von (43) mit Phenylethylamin und anschließender Acetylierung

0,14 mmol (72 mg) 2-[1-Hydroxy-4-methyl-3-(methyl-{3methyl-2-[(1-methyl-piperidine-2-carbonyl)-amino] -pentanoyl}-amino)-pentyl]-thiazol-4-carbonsäure-methylester (43) werden mit 100 µl Phenylethylamin versetzt und 12 h bei RT gerührt. Der gesamte Reaktionsansatz wird durch eine Kieselgelschicht filtriert und mit Ethylacetat nachgewaschen. Das Eluat wird komplett eingeengt und mit 40 µl Essigsäureanhydrid und 10 µl Pyridin versetzt. Man lässt die Reaktionsmischung 2 h bei RT rühren. Anschließend wird ein Drittel des Reaktionsansatzes auf der analytischen HPLC gereinigt.

### Referenzbeispiele 44 and 45:

### 1-Methyl-piperidin-2-carbonsäure-[1-({1-[2-hydroxy-2-(4-phenethylcarbamoyl-thiazol-2-yl)-ethyl]-2-methyl-propyl}methyl-carbamoyl)-2-methyl-butyl]-amid (44)

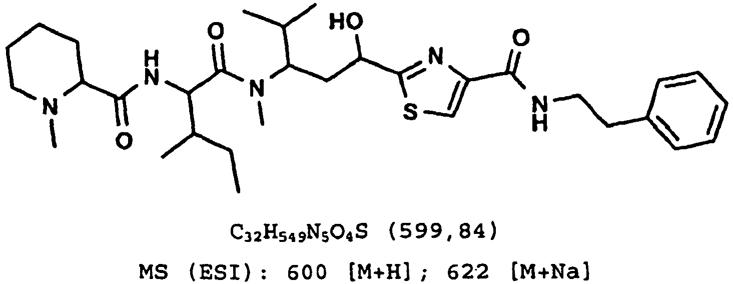

### Essigsäure-4-methyl-3-(methyl-{3-methyl-2-[(1-methyl-piperidine-2-carbonyl)-amino]-pentanoyl}-amino)-1-(4-phenethylcarbamoyl-thiazol-2-yl)-pentylester (45)

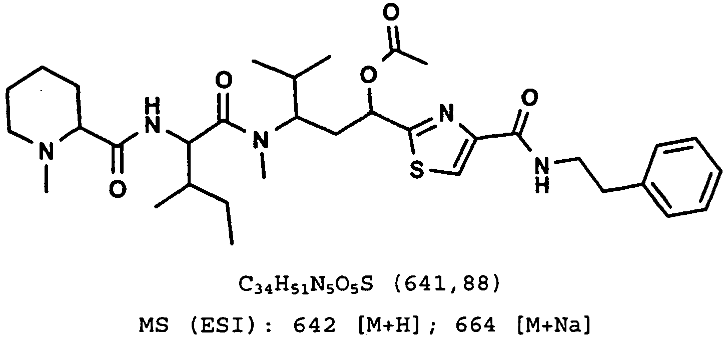

### Synthese von Baustein (VI) mit einer Evans-Synthese:

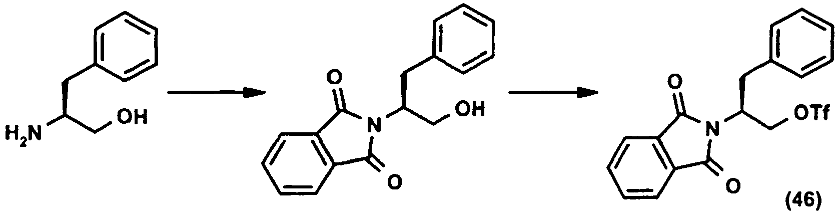

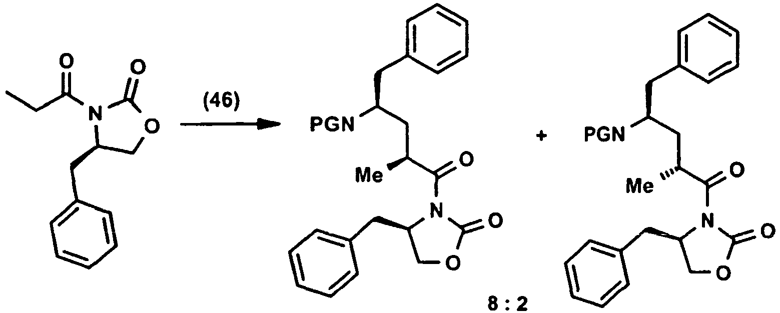

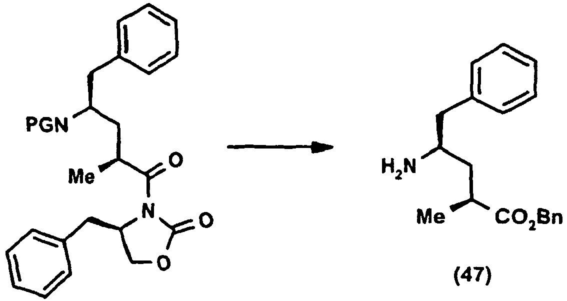

(2*S*)-2-Phthalimido-3-phenyl-propanol: Zu einem Gemisch aus *L*-Phenylalaninol (1.0 g, 6.61 mmol) und Na₂CO₃ (1.05 g, 9.92 mmol) in einer 1:1 Mischung aus THF (10 mL) und H₂O (10 mL) wurde *N*-Carbethoxyphthalimid (1.74 g, 7.94 mmol) gegeben und 4 h bei RT gerührt. Die Reaktionsmischung wurde mit EtOAc (20 mL) versetzt und die organische Phase abgetrennt. Die wassrige Phase wurde 2 mal mit je 15 mL EtOAc extrahiert und die vereinigten organischen Phasen mit ges. Kochsalzlösung gewaschen, über Na₂SO₄ getrocknet und einrotiert. Das gewünschte Produkt wurde mittels Säulenchromatographie mit 2% MeOH in CH₂Cl₂ gereinigt. Ausbeute: 1.41 g (76%); MS (ESI) 282 [M+H]; ¹H NMR (300 MHz, CDCl₃) : δ 7.82-7.76 (m, 2H), 7.73-7.66 (m, 2H), 7.24-7.12 (m, 5H), 4.70-4.58 (m, 1H), 4.12-4.02 (m, 1H), 3.98-3.88 (m, 1H), 3.20 (d, *J* = 12.5 Hz, 2H), 2.80-2.72 (m, 1H).

(2*S*)-1-Trifluoromethanesulfonyl-2-phthalimido-3-phenyl propanoat: Zu einer Lösung von (2S)-2-Phthalimido-3-phenylpropanol (0.42g, 1.49 mmol) in trockenem CH₂Cl₂ (5 mL) wurde bei -78 °C Pyridin (146 µL, 1.79 mmol) zugegeben und 20 min gerührt. Zu dieser Mischung wurde innerhalb von 3 min Trifluoromethansulfonsäure Anhydrid (264 µL, 1.57 mmol) gegeben und 1 h bei -78°C gerührt. Die Reaktionsmischung wurde mit 3 ml gesättigter Kochsalzlösung gequenched und die organische Phase abgetrennt. Die wässrige Phase wurde nochmals mit 5 mL of CH₂Cl₂ extrahiert, die vereinten organischen Phasen mit 5 ml gesättigter Kochsalzlösung gewaschen, über Na₂SO₄ getrocknet und einrotiert. Das gewünschte Produkt wurde mittels Säulenchromatographie mit 20% EtOAc in Hexan gereinigt. Ausbeute: 0.41 g (66%). MS (ESI) 414 [M+H];
¹H NMR (300 MHz, CDCl₃): δ 7.84-7.77 (m, 2H), 7.75-7.68 (m, 2H), 7.28-7.14 (m, 5H), 5.18 (t, *J* = 13.0 Hz, 1H), 5.00-4.85 (m, 1H), 4.55-4.30 (m, 1H), 3.40-3.25 (m, 2H).

Evans Alkylierung: Unter Argon wurde (4R)-3-propanoyl-4-benzyl-2-oxazolidinon (0.100 g, 0.43 mmol) in 2 ml trockenem THF gelöst und auf -40°C gekühlt. LiHMDS (1M/THF) (0.47 mL, 0.47 mmol) wurde zugegeben und 45 min gerührt. Anschliessend wurde (2S)-1-Trifluoromethan-sulfonyl-2-phthalimido-3-phenylpropanoat (0.266 g, 0.64 mmol) in trockenem THF (2 mL) zugegeben. Die Reaktionsmischung wurde 4h bei -40°C gerührt und anschliessend durch Zugabe von 3 ml gesättigter Kochsalzlösung gequenched. Die organische Phase wurde abgetrennt und die wässrige Phase 2 mal mit je 5 ml EtOAc extrahiert. Die vereinten organischen Phasen wurden mit 3 ml gesättigter Kochsalzlösung gewaschen, über Na₂SO₄ getrocknet und einrotiert. Das gewünschte Produkt wurde mittels Säulenchromatographie mit 25% EtOAc in Hexan gereinigt. Ausbeute: 0.149 g (70%). Die Diastereomeren wurden mittels präparativer Dünnschichtchromatographie getrennt, wobei das gewünschte Produkt mit einem Überschuss von 8:2 erhalten wurde.

### (2'S, 4'R, 4R,)-3-(2'Methyl-4'phthalimido-5'phenyl pentanoyl)-4-benzyl-1,3-oxazolidin-2-on (Hauptprodukt):

MS (ESI): 497 [M+H]; ¹H NMR (300 MHz, CDCl₃) : δ 7.77 (t, *J* = 8.5 Hz, 2H), 7.63 (t, *J* = 8.4 Hz, 1H), 7.55 (t, *J* = 8.4 Hz, 1H), 7.42 (d, *J* = 8.5 Hz, 2H), 7.37-7.22 (m, 6H), 7.10 (d, *J*= 8.6 Hz, 2H), 5.08 (q, *J* = 9.6 and 16.1 Hz, 1H), 4.56-4.42 (m, 2H), 4.20-4.00 (m, 4H), 3.45 (dd, *J* = 10.7 and 16.1 Hz, 1H), 3.12-2.98 (m, 2H), 2.34 (dd, *J* = 12.8 and 13.9 Hz, 1H), 1.62 (d, *J* = 8.6 Hz, 3H).

### (2'R, 4'R, 4R,)-3-(2'Methyl-4'phthalimido-5'phenyl pentanoyl)-4-benzyl-1,3-oxazolidin-2-on (Nebenprodukt):

MS (ESI) : 497 [M+H]; ¹H NMR (300 MHz, CDCl₃) : δ 8.12 (d, *J* = 8.6 Hz, 1H), 7.76 (d, *J* = 8.5 Hz, 1H), 7.63 (t, *J* = 8.6 Hz, 1H), 7.53 (t, *J* = 8.5 Hz, 1H), 7.40-7.20 (m, 10H), 5.10 (q, *J* = 7.5 and 15.0 Hz, 1H), 4.94-4.84 (m, 1H), 4.54-4.42 (m, 1H), 4.36-4.08 (m, 4H), 3.46-3.30 (m, 2H), 3.12 (dd, *J* = 9.6 and 11.8 Hz, 1H), 2.88 (dd, *J* = 9.5 and 12.8 Hz, 1H), 1.00 (d, *J* = 9.6 Hz, 3H).

Abspaltung des Oxazolidinons: Evans et. al., J. Am. Chem. Soc. 1982, 104, 1737-1739.

Entschützung des Phthalimids: mittels Hydrazine/EtOH bei Raumtemperatur: Sasaki, T. et. al., J. Org. Chem. 1978, 43, 2320; Khan, M. N. et. al., J. Org. Chem. 1995, 60, 4536.

Mit den hier beschriebenen Synthesevorschriften wurden ausserdem noch weitere Tubulysinderivate als Referenz beispiele hergestellt:

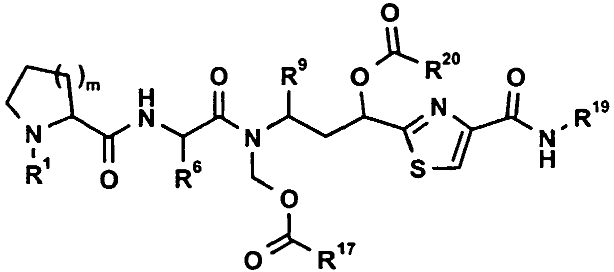

Dabei wurden folgende Reste verwendet:
- m =: 0, 1, 2, 3;
- R¹ =: Methyl, Ethyl;
- R⁶ =: Isopropyl, Isobutyl, Ethyl, Cyclopropyl, CH₂-Cyclo-propyl, CH (CH₃) CH₂CH₃;
- R⁹ =: Isopropyl, Trifluormethyl, Chlormethyl, Isobutyl, Ethyl, Cyclopropyl, CH₂-Cyclopropyl, CH(CH₃)CH₂CH₃, Cyclopentyl, Cyclohexyl;
- R¹⁷ =: Methyl, Ethyl, Propyl, Isopropyl, Butyl, Isobutyl, CH=C(CH₃), Cyclopropyl, Cyclobutyl, Cyclohexyl;
- R²⁰ =: Methyl, Ethyl, Propyl, Isopropyl, Phenyl;
- R¹⁹ =:

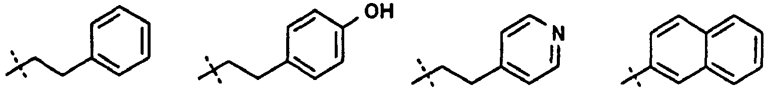

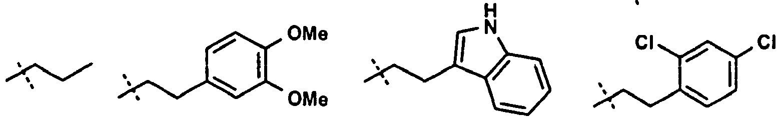

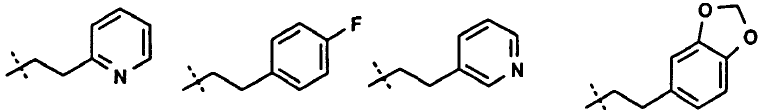

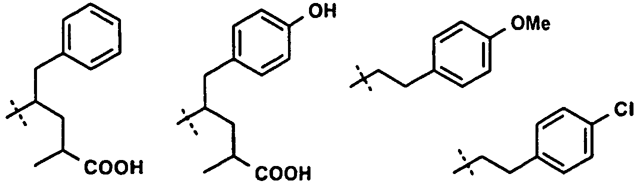

## Patentansprüche

1. Verbindungen der allgemeinen Formel 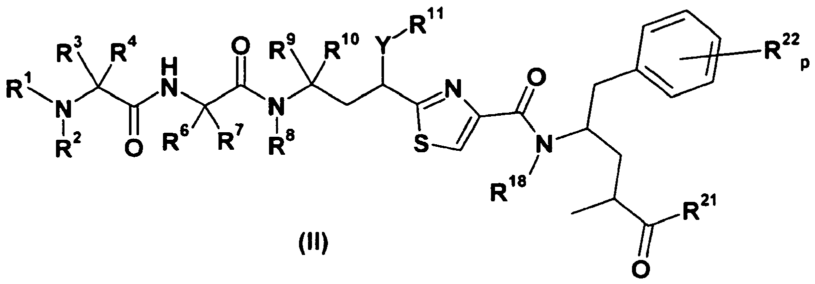 wobei
Y ein Sauerstoffatom, ein Schwefelatom oder eine Gruppe der Formel NR¹⁶ ist, wobei R¹⁶ ein Wasserstoffatom, ein Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest ist;
R¹ eine C₁-C₄ Alkylgruppe ist;
R² und R³ zusammen eine Gruppe der Formel (CH₂)ₙ mit n gleich 2, 3, 4 oder 5 sind;
R⁴ ein Wasserstoffatom oder eine Methylgruppe ist;
R⁶ eine C₁-C₆ Alkyl-, eine C₃-C₆ Cycloalkyl oder eine C₄-C₇ Alkylcycloalkylgruppe ist;
R⁷ ein Wasserstoffatom oder eine Methylgruppe ist;
R⁸ ein Wasserstoffatom, ein Alkyl-, Alkenyl- oder ein Aralkylrest ist;
R⁹ eine C₁-C₆ Alkylgruppe ist;
R¹⁰ ein Wasserstoffatom oder eine Methylgruppe ist;
R¹¹ ein Wasserstoffatom, ein Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest ist;
R¹⁸ ein Wasserstoffatom oder eine Methylgruppe ist;
R²¹ eine OH, NH₂, Alkyloxy-, Alkylamino oder eine Dialkylaminogruppe ist;
R²² ein Halogenatom, eine OH, NO₂, NH₂, Alkyloxy-, Alkylamino oder eine Dialkylaminogruppe ist und
p gleich 0, 1, 2 oder 3 ist,
oder ein pharmakologisch akzeptables Salz, Solvat, Hydrat oder eine pharmakologisch akzeptable Formulierung derselben, wobei die folgenden Definitionen gelten:
Der Ausdruck Alkyl bezieht sich auf eine gesättigte, geradkettige oder verzweigte Kohlenwasserstoffgruppe, die 1 bis 20 Kohlenstoffatome aufweist.
Die Ausdrücke Alkenyl und Alkinyl beziehen sich auf zumindest teilweise ungesättigte, geradkettige oder verzweigte Kohlenwasserstoffgruppen; die 2 bis 20 Kohlenstoffatome aufweisen.
Des weiteren beziehen sich die Begriffe Alkyl, Alkenyl und Alkinyl auf Gruppen, bei der ein oder mehrere Wasserstoffatome durch ein Halogenatom ersetzt sind.
Der Ausdruck Heteroalkyl bezieht sich auf eine Alkyl-, eine Alkenyl- oder eine Alkinyl-Gruppe, in der ein oder mehrere Kohlenstoffatome durch ein Sauerstoff-, Stickstoff-, Phosphor-, Bor-, Selen-, Silizium- oder Schwefelatom ersetzt sind. Der Ausdruck Heteroalkyl bezieht sich des weiteren auf eine Carbonsäuregruppe oder eine von einer Carbonsäure abgeleitete Gruppe.
Der Ausdruck Cycloalkyl bezieht sich auf eine gesättigte oder teilweise ungesättigte cyclische Gruppe, die einen oder mehrere Ringe aufweist, die insgesamt 3 bis 14 Ring-Kohlenstoffatome enthalten. Der Ausdruck Cycloalkyl bezieht sich weiterhin auf derartige Gruppen, bei denen ein oder mehrere Wasserstoffatome durch Fluor-, Chlor-, Brom- oder Jodatome oder OH, =O, SR, =S, NH₂, =NH oder NO₂-Gruppen ersetzt sind.
Der Ausdruck Heterocycloalkyl bezieht sich auf eine Cycloalkylgruppe wie oben definiert, in der ein oder mehrere Ring-Kohlenstoffatome durch ein Sauerstoff-, Stickstoff-, Silizium-, Selen-, Phosphor- oder Schwefelatom ersetzt sind. Der Ausdruck Heterocycloalkyl bezieht sich weiterhin auf derartige Gruppen, bei denen ein oder mehrere Wasserstoffatome durch Fluor-, Chlor-, Brom- oder Jodatome oder OH, =O, SH, =S, NH₂, =NH oder NO₂-Gruppen ersetzt sind.
Der Ausdruck Alkylcycloalkyl bezieht sich auf Gruppen, die entsprechend den obigen Definitionen sowohl Cycloalkyl- wie auch Alkyl-, Alkenyl- oder Alkinylgruppen enthalten.
Der Ausdruck Heteroalkylcycloalkyl bezieht sich auf Alkylcycloalkylgruppen, wie oben definiert, in der ein oder mehrere Kohlenstoffatome durch ein Sauersoff-, Stickstoff-, Silizium-, Selen-, Phosphor- oder Schwefelatom ersetzt sind.
Der Ausdruck Aryl bezieht sich auf eine aromatische Gruppe, die einen oder mehrere Ringe hat, welche insgesamt 6 bis 14 Ring-Kohlenstoffatome enthalten. Der Ausdruck Aryl bezieht sich weiterhin auf derartige Gruppen, bei denen ein oder mehrere Wasserstoffatome durch Fluor-, Chlor-, Brom- oder Jodatome oder OH, SH, NH₂, oder NO₂-Gruppen ersetzt sind.
Der Ausdruck Heteroaryl bezieht sich auf eine aromatische Gruppe, die einen oder mehrere Ringe hat, welche insgesamt 5 bis 14 Ringatome enthalten und ein oder mehrere Sauerstoff-, Stickstoff-, Phosphor- oder Schwefel-Ringatome enthält. Der Ausdruck Heteroaryl bezieht sich weiterhin auf derartige Gruppen, bei denen ein oder mehrere Wasserstoffatome durch Fluor-, Chlor-, Brom- oder Jodatome oder OH, SH, NH₂, oder NO₂-Gruppen ersetzt sind.
Der Ausdruck Aralkyl bezieht sich auf Gruppen, die entsprechend den obigen Definitionen sowohl Aryl- wie auch Alkyl-, Alkenyl-, Alkinyl- und/oder Cycloalkylgruppen enthalten. Der Ausdruck Heteroaralkyl bezieht sich auf eine Aralkylgruppe wie oben definiert, in der ein oder mehrere Kohlenstoffatome durch ein Sauerstoff-, Stickstoff-, Silizium-, Selen-, Phosphor-, Bor- oder Schwefelatom ersetzt sind, d. h. auf Gruppen, die entsprechend den obigen Definitionen sowohl Aryl- bzw. Heteroaryl- wie auch Alkyl-, Alkenyl-, Alkinyl- und/oder Heteroalkyl- und/oder Cycloalkyl- und/oder Heterocycloalkylgruppen enthalten.
Die Ausdrücke Cycloalkyl, Heterocycloalkyl, Alkylcycloalkyl, Heteroalkylcycloalkyl, Aryl, Heteroaryl, Aralkyl und Heteroaralkyl beziehen sich auch auf Gruppen, in denen ein oder mehrere Wasserstoffatome solcher Gruppen durch Fluor-, Chlor-, Brom- oder Jodatome oder OH, =O SH, =S, NH₂, =NH oder NO₂-Gruppen ersetzt sind,
wobei die folgende Verbindung ausgenommen ist:

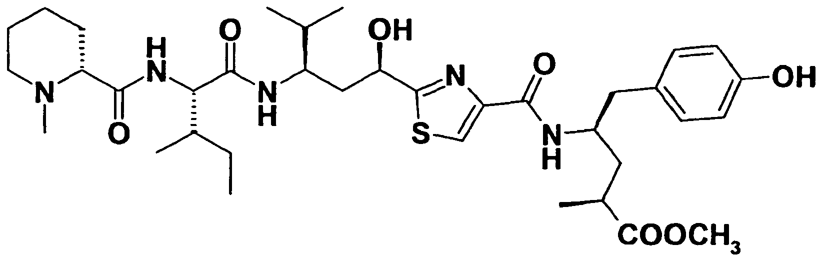

2. Verbindungen nach Anspruch 1, wobei Y ein Sauerstoffatom ist.

3. Verbindungen nach Anspruch 1 oder 2, wobei R⁶ der Seitenkette von Leucin, Isoleucin oder Valin entspricht.

4. Verbindungen nach einem der Ansprüche 1 bis 3, wobei R¹¹ ein Wasserstoffatom oder eine Gruppe der Formel -(C=O)-(C₁₋₄)Alkyl ist.

5. Pharmazeutische Zusammensetzung, die eine Verbindung nach einem der Ansprüche 1 bis 4 und fakultativ Trägerstoffe und/oder Adjuvanzien enthält.

6. Verbindung oder pharmazeutische Zusammensetzung nach einem der Ansprüche 1 bis 5 zur Anwendung in der Behandlung von Tumorerkrankungen, immunologisch bedingten Krankheiten, Autoimmunkrankheiten, entzündlichen Erkrankungen und rheumatoider Arthritis sowie zur Oberflächenmodifikation von Kunststoff- und Metallimplantaten.

7. Verbindung oder pharmazeutische Zusammensetzung nach einem der Ansprüche 1 bis 5 zur Anwendung in der Behandlung von Krebserkrankungen.

## Claims

1. Compounds of the general formula 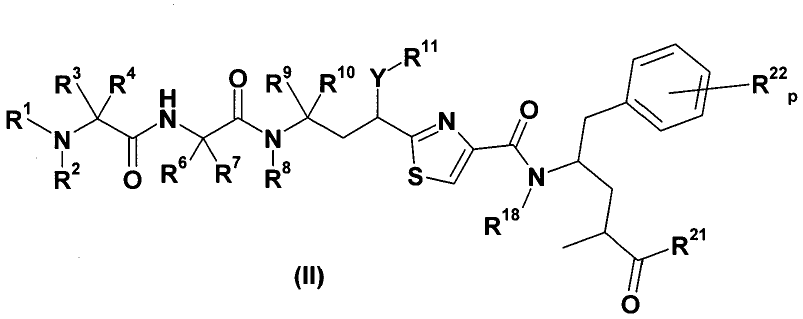 wherein
Y is an oxygen atom, a sulfur atom or a group of Formula NR¹⁶ wherein R¹⁶ is a hydrogen atom, an alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl residue;
R¹ is a C₁-C₄ alkyl group;
R² and R³ together are a group of formula (CH₂)ₙ wherein n is 2, 3, 4 or 5;
R⁴ is a hydrogen atom or a methyl group;
R⁶ is a C₁-C₆ alkyl, a C₃-C₆ cycloalkyl or a C₄-C₇ alkylcycloalkyl group;
R⁷ is a hydrogen atom or a methyl group;
R⁸ is a hydrogen atom, an alkyl, alkenyl or an aralkyl residue;
R⁹ is a C₁-C₆ alkyl group;
R¹⁰ is a hydrogen atom or a methyl group;
R¹¹ is a hydrogen atom, an alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl residue;
R¹⁸ is a hydrogen atom or a methyl group;
R²¹ is an OH, NH₂, alkyloxy, alkylamino or a dialkylamino group;
R²² is a halogen atom, an OH, NO₂, NH₂, alkyloxy, alkylamino or a dialkylamino group and
p is 0, 1, 2 or 3,
or a pharmacologically acceptable salt, solvate, hydrate or a pharmacologically acceptable formulation thereof, wherein the following definitions apply:
The term alkyl refers to a saturated, straight-chain or branched hydrocarbon group, containing from one to twenty carbon atoms.
The term alkenyl and alkynyl refer to a at least partially unsaturated, straight-chain or branched hydrocarbon groups, containing from two to twenty carbon atoms.
Furthermore the terms akyl, alkenyl and alkynyl refer to groups wherein one or more hydrogen atoms are replaced by a halogen atom.
The term heteroalkyl refers to an alkyl, alkenyl or alkynyl group, wherein one or more carbon atoms are replaced by an oxygen, nitrogen, phosphorus, boron, selenium, silicon or sulphur atom. The term heteroalkyl furthermore refers to a carboxylic acid or a group derived from a carboxylic acid.
The term cycloalkyl refers to a saturated or partially unsaturated cyclic group that contains one or more rings, which contain a total of three to fourteen ring carbon atoms. The term cycloalkyl furthermore refers to such groups in which one or more hydrogen atoms have been replaced by fluorine, chlorine, bromine or iodine atoms or by OH, =O, SH, =S, NH₂, =NH or NO₂ groups.
The term heterocycloalkyl refers to a cycloalkyl group as defined above in which one or more ring carbon atoms have been replaced by an oxygen, nitrogen, silicon, selenium, phosphorus or sulphur atom. The term heterocycloalkyl furthermore refers to such groups in which one or more hydrogen atoms have been replaced by fluorine, chlorine, bromine or iodine atoms or by OH, =O, SH, =S, NH₂, =NH or NO₂ groups.
The term alkylcycloalkyl refers to groups that contain both cycloalkyl and also alkyl, alkenyl or alkynyl groups in accordance with the above definitions.
The term heteroalkylcycloalkyl refers to alkylcycloalkyl groups as defined above in which one or more carbon atoms have been replaced by an oxygen, nitrogen, silicon, selenium, phosphorus or sulphur atom.
The term aryl refers to an aromatic group having one or more rings containing a total of 6 to 14 ring carbon atoms. The term aryl furthermore refers to such groups in which one or more hydrogen atoms have been replaced by fluorine, chlorine, bromine or iodine atoms or by OH, SH, NH₂ or NO₂ groups.
The term heteroaryl refers to a aromatic group having one or more rings, comprising a total of five to fourteen ring atoms, and one or more oxygen, nitrogen, phosphorous or sulfur ring atoms. The term heteroaryl furthermore refers to such groups in which one or more hydrogen atoms have been replaced by fluorine, chlorine, bromine or iodine atoms or by OH, SH, NH₂ or NO₂ groups.
The term aralkyl refers to groups containing both aryl and also alkyl, alkenyl, alkynyl and/or cycloalkyl groups in accordance with the above definitions.
The term heteroaralkyl refers to an aralkyl group as defined above, wherein one or more carbon atoms have been replaced by an oxygen, nitrogen, silicon, selenium, phosphorus, boron or sulfur atom, i.e. to groups containing both aryl or heteroaryl, respectively, and also alkyl, alkenyl, alkynyl and/or heteroalkyl and/or cycloalkyl and/or heterocycloalkyl groups in accordance with the above definitions.
The terms cycloalkyl, heterocycloalkyl, alkylcycloalkyl, heteroalkylcycloalkyl, aryl, heteroaryl, aralkyl and heteroaralkyl also refer to groups wherein one or more hydrogen atoms of such groups have been replaced by fluorine, chlorine, bromine or iodine atoms or by OH, =O, SH, =S, NH₂, =NH or NO₂ groups,
wherein the following compound is excluded:

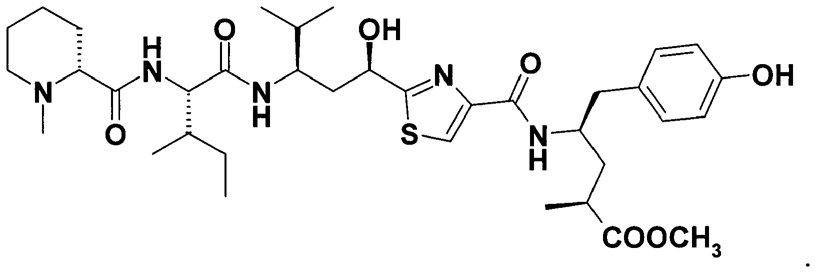

2. Compounds according to claim 1, wherein Y is an oxygen atom.

3. Compounds according to claim 1 or 2, wherein R⁶ corresponds to the side chain of leucine, isoleucine or valine.

4. Compounds according to any one of claims 1 to 3, wherein R¹¹ is a hydrogen atom or a group of formula - (C=O) - (C₁₋₄)alkyl.

5. A pharmaceutical composition comprising a compound according to any one of claims 1 to 4 and optionally one or more carriers and/or adjuvants.

6. Compound or pharmaceutical composition according to any one of claims 1 to 5 for use in the treatment of tumor diseases, immunologically caused diseases, autoimmune diseases, inflammatory diseases and rheumatoid arthritis as well as for the surface treatment of plastic and metal implants.

7. Compound or pharmaceutical composition according to any one of claims 1 to 5 for use in the treatment of cancer.

## Revendications

1. Composés de formule générale: 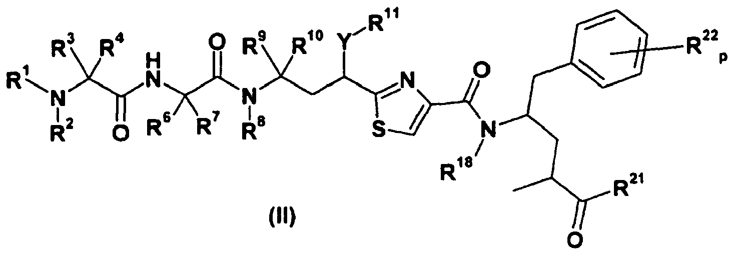 où
Y est un atome d'oxygène, un atome de soufre ou un groupe de formule NR¹⁶, R¹⁶ étant un atome d'hydrogène, un groupement alkyle, un groupement alcényle, un groupement alcinyle, un groupement hétéroalkyle, un groupement aryle, un groupement hétéroaryle, un groupement cycloalkyle, un groupement alkylcycloalkyle, un groupement hétéroalkylcycloalkyle, un groupement hétérocycloalkyle, un groupement aralkyle ou un groupement hétéroaralkyle;
R¹ est un groupe alkyle en C₁-C₄;
R² et R³ constituent ensemble un groupe de formule (CH₂)ₙ, avec n étant égal à 2, 3, 4 ou 5;
R⁴ est un atome d'hydrogène ou un groupe méthyle;
R⁶ est un groupe alkyle en C₁-C₆, un groupe cycloalkyle en C₃-C₆ ou un groupe alkylcycloalkyle en C₄-C₇;
R⁷ est un atome d'hydrogène ou un groupe méthyle ;
R⁸ est un atome d'hydrogène, un groupement alkyle, un groupement alcényle ou un groupement aralkyle;
R⁹ est un groupe alkyle en C₁-C₆;
R¹⁰ est un atome d'hydrogène ou un groupe méthyle ;
R¹¹ est un atome d'hydrogène, un groupement alkyle, un groupement alcényle, un groupement alcinyle, un groupement hétéroalkyle, un groupement aryle, un groupement hétéroaryle, un groupement cycloalkyle, un groupement alkyl-cycloalkyle, un groupement hétéroalkylcycloalkyle, un groupement hétérocycloalkyle, un groupement aralkyle ou un groupement hétéroaralkyle;
R¹⁸ est un atome d'hydrogène ou un groupe méthyle ;
R²¹ est un groupe OH, un groupe NH₂, un groupe alkyloxy, un groupe alkylamine ou un groupe dialkylamine;
R²² est un atome d'halogène, un groupe OH, un groupe NO₂, un groupe NH₂, un groupe alkyloxy, un groupe alkylamine ou un groupe dialkylamine et
p est égal à 0, 1, 2 ou 3,
ou un sel, un produit de solvatation, un hydrate pharmaceutiquement acceptable ou une formulation pharmaceutiquement acceptable de ceux-ci, où les définitions suivantes sont valables:
L'expression alkyle se rapporte à un groupe hydrocarboné saturé, linéaire ou branché, qui présente de 1 à 20 atomes de carbone.
Les expressions alcényle et alcinyle se rapportent à des groupes hydrocarbonés au moins partiellement insaturés, à chaîne linéaire ou branchée, qui présentent de 2 à 20 atomes de carbone.
En outre, les expressions alkyle, alcényle et alcinyle se rapportent à des groupes dans lesquels un ou plusieurs atomes d'hydrogène sont remplacés par un atome d'halogène.
L'expression hétéroalkyle se rapporte à un groupe alkyle, un groupe alcényle ou un groupe alcinyle dans lequel un ou plusieurs atomes de carbone sont remplacés par un atome d'oxygène, un atome d'azote, un atome de phosphore, un atome de bore, un atome de sélénium, un atome de silicium ou un atome de soufre. L'expression hétéroalkyle se rapporte en outre à un groupe acide carboxylique ou un groupe dérivé d'un acide carboxylique.
L'expression cycloalkyle se rapporte à un groupe cyclique saturé ou partiellement insaturé qui présente un ou plusieurs cycles qui contiennent au total de 3 à 14 atomes de carbone cycliques. L'expression cycloalkyle se rapporte en outre à de tels groupes, dans lesquels, un ou plusieurs atomes d'hydrogène sont remplacés par des atomes de fluor, des atomes de chlore, des atomes de brome ou des atomes d'iode ou des groupes OH, des groupes =O, des groupes SH, des groupes =S, des groupes NH₂, des groupes =NH ou des groupes NO₂.
L'expression hétérocycloalkyle se rapporte à un groupe cycloalkyle, tel que défini ci-dessus, dans lequel un ou plusieurs atomes de carbone cycliques sont remplacés par un atome d'oxygène, un atome d'azote, un atome de silicium, un atome de sélénium, un atome de phosphore ou un atome de soufre. L'expression hétérocycloalkyle se rapporte en outre à de tels groupes, dans lesquels un ou plusieurs atomes d'hydrogène sont remplacés par des atomes de fluor, des atomes de chlore, des atomes de brome ou des atomes d'iode ou des groupes OH, des groupes =O, des groupes SH, des groupes =S, des groupes NH₂, des groupes =NH ou des groupes NO₂.
L'expression alkylcycloalkyle se rapporte à des groupes qui contiennent, conformément aux définitions ci-dessus, aussi bien des groupes cycloalkyles que des groupes alkyles, des groupes alcényles ou des groupes alcinyles.
L'expression hétéroalkylcycloalkyle se rapporte à des groupes alkylcycloalkyles, tels que définis ci-dessus, dans lesquels un ou plusieurs atomes de carbone sont remplacés par un atome d'oxygène, un atome d'azote, un atome de silicium, un atome de sélénium, un atome de phosphore ou un atome de soufre.
L'expression aryle se rapporte à un groupe aromatique qui possède un ou plusieurs cycles qui contiennent au total de 6 à 14 atomes de carbone cycliques. L'expression aryle se rapporte en outre à de tels groupes dans lesquels un ou plusieurs atomes d'hydrogène sont remplacés par des atomes de fluor, des atomes de chlore, des atomes de brome ou des atomes d'iode ou des groupes OH, des groupes SH, des groupes NH₂, ou des groupes NO₂.
L'expression hétéroaryle se rapporte à un groupe aromatique qui possède un ou plusieurs cycles qui contiennent au total de 5 à 14 atomes cycliques et un ou plusieurs atomes d'oxygène, d'azote, de phosphore ou de soufre par cycle. L'expression hétéroaryle se rapporte en outre à de tels groupes dans lesquels un ou plusieurs atomes d'hydrogène sont remplacés par des atomes de fluor, des atomes de chlore, des atomes de brome ou des atomes d'iode ou des groupes OH, des groupes SH, des groupes NH₂, ou des groupes NO₂.
L'expression aralkyle se rapporte à des groupes, qui, conformément aux définitions ci-dessus, contiennent aussi des groupes aryle que des groupes alkyles, des groupes alcényles, des groupes alcinyles et/ou des groupes cycloalkyles.
L'expression hétéroaralkyle se rapporte à un groupe aralkyle, tel que défini ci-dessus, dans lequel un ou plusieurs atomes de carbone sont remplacés par un atome d'oxygène, un atome d'azote, un atome de silicium, un atome de sélénium, un atome de phosphore, un atome de bore ou un atome de soufre, c'est-à-dire à des groupes, qui conformément aux définitions ci-dessus, contiennent aussi bien des groupes aryles, respectivement des groupes hétéroaryles, que des groupes alkyles, des groupes alcényles, des groupes alcinyles et/ou des groupes hétéroalkyles et/ou des groupes cycloalkyles et/ou des groupes hétérocycloalkyles.
Les expressions cycloalkyle, hétérocycloalkyle, alkylcycloalkyle, hétéroalkylcycloalkyle, aryle, hétéroaryle, aralkyle et hétéroaralkyle se rapportent également à des groupes dans lesquels un ou plusieurs atomes d'hydrogène de tels groupes sont remplacés par des atomes de fluor, des atomes de chlore, des atomes de brome ou des atomes d'iode ou des groupes OH, des groupes =O, des groupes SH, des groupes =S, des groupes NH₂, des groupes =NH ou des groupes NO₂,
le composé suivant étant exclu:

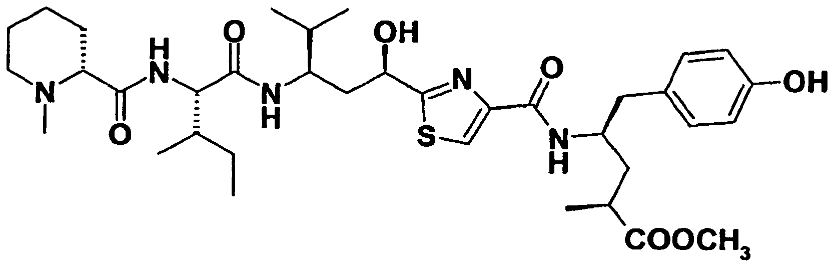

2. Composés selon la revendication 1 où Y est un atome d'oxygène.

3. Composés selon les revendications 1 ou 2, le groupe R⁶ de la chaîne latérale correspondant à de la leucine, à de l'isoleucine ou à de la valine.

4. Composés selon l'une des revendications 1 à 3, le groupe R¹¹ étant un atome d'hydrogène ou un groupe de formule -(C=O)-alkyle en (C₁₋₄).

5. Composition pharmaceutique qui contient un composé selon l'une des revendications 1 à 4 et facultativement des substances véhicules et/ou des adjuvants.

6. Composé ou composition pharmaceutique selon l'une des revendications de 1 à 5 pour une utilisation dans le traitement de maladies tumorales, de maladies entraînées par immunologie, de maladies auto-immunes, de maladies inflammatoires et de l'arthrite rhumatoïde, ainsi que pour une modification de surface d'implants en matière synthétique et en métal.

7. Composé ou composition pharmaceutique selon l'une des revendications de 1 à 5 pour une utilisation dans le traitement de maladies cancéreuses.
